# EUROPEAN PATENT SPECIFICATION

(11) **EP 3 315 461 B1**
(45) Date of publication and mention of the grant of the patent: **07.07.2021**
(21) Application number: 16813709.9
(22) Date of filing: 23.06.2016
(51) Int. Cl.: B81B 7/02, C12Q 1/68, G01N 33/487, C12Q 1/6869

(54) **MICRO-POROUS ELECTRODE AND METHOD FOR ANALYSIS OF CHEMICAL SUBSTANCES**
MIKROPORÖSE ELEKTRODE UND VERFAHREN ZUR ANALYSE CHEMISCHER SUBSTANZEN
MICRO-ÉLECTRODE POREUSE ET PROCÉDÉ D'ANALYSE DE SUBSTANCES CHIMIQUES

(30) Priority: 23.06.2015 CN 201510348809; 23.06.2015 US 201562183617 P
(43) Date of publication of application: 02.05.2018
(73) Proprietor: BGI Shenzhen, Shenzhen, Guangdong 518083 (CN); BGI Shenzhen Co., Limited, Shenzhen, Guangdong 518083 (CN)
(72) Inventor: LI, Handong, San Jose, California 95129 (US); LIN, Jianxun, Shenzhen, Guangdong 518083 (CN); YUN, Quanxin, Shenzhen, Guangdong 518083 (CN); XIANG, Shaohua, Shenzhen, Guangdong 518083 (CN); DRMANAC, Radoje, Los Altos Hill, CA 94022 (US); DRMANAC, Snezana, Los Altos Hill, CA 94022 (US); ZHANG, Yongwei, Saratoga, CA 95070 (US)
(74) Representative: Inspicos P/S
(86) International application number: PCT/CN2016/086846
(87) International publication number: WO 2016/206593

(56) References cited:
- EP-A2- 2 652 153
- WO-A1-00/70073
- WO-A2-2010/044932
- WO-A2-2011/082419
- WO-A2-2013/154999
- CN-A- 101 848 757
- CN-A- 102 095 768
- CN-A- 103 193 189
- CN-A- 103 424 457
- CN-A- 104 350 162
- US-A1- 2003 040 173
- US-A1- 2012 097 539
- US-A1- 2013 109 577
- US-B2- 8 500 979

## Description

### FIELD OF THE INVENTION

The present invention relates to the field of semiconductor technology and chemical substance analysis, and more particularly to a microwell electrode and a method for manufacturing the same, a microwell electrode array, a sensor chip and a sequencing system, and a method for analysis of a chemical substance and a nucleic acid molecule based on the microwell electrode, the microwell electrode array, the sensor chip, or the sequencing system.

### BACKGROUND

The $1,000 genome era is approachingto us thanks to the continuous improvement of the second-generation DNA sequencing technology over the past decade. However, to put $1,000 genome sequencing into effect and push the application of DNA sequencing technology in personalized medicine, substantial development is still needed. Here are four major problems that the second-generation sequencing platforms are facing: (1) short read length due to the inherent dephasing; (2) slow read rate due to the elution step required for base incorporation; (3) laborious and costly sample preparation required for amplification; (4) expensive optical system. Single molecule sequencing technology is considered as the most promising approach to address all the above problems simultaneously.

A new sequencing technology is nanopore-based sequencing technology. The basis idea of this technology lies in that, when an individual DNA molecule is passing through a nanopore, this nanopore structure serves as both a restriction site and an incorporation site simultaneously. Oxford Nanopore Technology (ONT), a leader in nanopore sequencing, has recently released its first protein nanopore sequencer with a read length of 10,000 bases and a read speed of 100 bases per second. Compared with Pacific Biosciences (PacBio), a leader in single molecule sequencing, the technique of ONT can bring down the cost and size of sequencer significantly as no optical device is required.

The development of protein nanopore-based sequencing technique is faster as compared to solid-state nanopore, even though it is a general consensus that solid-state nanopore is much more superior in terms of stability and scalability, which are extremely important for a robust and low-cost sequencing device. A solid-state nanopore still lacks atomic precision and chemical specificity compared with a protein nanopore. The chemical specificity can be generally met with various surface modification techniques. However, reproducible production of large nanopore arrays still faces difficulty in its manufacturing process. Most of the current nanopore-based sequencing methods rely on 3D nanoscale-size structure, not only the diameter of a pore should be small enough, but also the thickness of the pore or a electrode should be as small as the distance between adjacent bases. The most common method to form such a nanopore is to etch with an ion beam or drill with an electron beam on a thin insulating material such as silicon nitride or graphene. However, this method is unconventional and not compatible with the standard semiconductor manufacturing process. This makes nanopore manufacturing process very costly and unreproducible. While from the point of commercial application, nanopore-based technique can offer very long read length that is superior to other current techniques, and can greatly reduce sequencing costs.

There are two approaches to circumvent the requirements for accurate and effective manufacturing process required for solid-state nanopore. One approach is to modify DNA sample to improve a signal difference among bases, thus relieving the requirements for accuracy and size of nanopore. There are some nanopore-based sequencing companies, such as Genia Technologies and Stratos Genomics, currently pursuing this approach. For example, in Stratos Genomics' sequencing with amplification technology, each base is replaced with a large reporter molecule with a strong signal using a proprietary molecular amplification method. Genia Technologies uses DNA polymerase to sequence a template DNA, as base-specific tags produced by enzymatic cleavage can be captured and recognized by a nanopore. Although these companies are currently focusing on protein nanopore, the same idea can be applied to solid-state nanopore without any technical obstacle. The other approach is to simplify 3D nanostructure to 2D nanostructure, since the standard semiconductor fabrication process has been applicable for reuglar 2D nanostructures, such as nanowire, nanochannel, and nanogap. The challenge has become how to achieve single base resolution while the number of one-dimensional nanostructure is no longer limited to a single digit. There are already a few approaches to address this issue. Nabsys is developing a positional sequencing platform that generates a long-distance sequencing map with short probe sequences by detecting sequence-specific tags bound to a DNA template when the DNA template passs through a nanochannel (∼100 nm).

In conclusion, nanopore-based DNA sequencing still faces many technical challenges. Therefore, how to overcome the above problems and propose a low-cost and high-throughput nanopore-based DNA sequencing scheme has become the focus of science and technology around the world. The development of a new generation of DNA detection technology at single molecule level with independent intellectual property rights will play an important role in the layout of China's high-tech industry in the future. Moreover, the integration and portability of the solution of the above problems will have a positive promotion effect on many fields, such as disease diagnosis, food testing and environmental monitoring.

US 2012/097539 A1 provides a nanoparticle translocation device including a first reservoir having a first reservoir electrode, a second reservoir having a second reservoir electrode, and at least one nanopore providing fluid communication between the first and second reservoirs. The device also includes one or more inner electrode portions on an inner wall of the nanopore and one or more outer electrode portions disposed on an outer wall of the nanopore.

US 8500979 B2 provdides an electronic sensor having at least two electrodes embedded in a nonconducting layer and separated by a nanoscale gap. The separation between the first electrode and the second electrode forms a cavity capable of containing a fluid. Two or more posts comprised of an insulating material extend into the cavity from the face of the first electrode to the face of the second electrode.

WO 2011/082419 A2 provdides an analyte detection system comprising: a nanochannel having a first end, a second end opposite the first end, a first side, and a second side opposite the first side; a pair of electrophoretic electrodes, comprising a first electrophoretic electrode at the first end and a second electrophoretic electrode at the second end; a pair of orthogonal electrodes, comprising a first orthogonal electrode at first side and a second orthogonal electrode at the second side; and a plurality of nano-field effect transistor devices (nanoFETs) disposed in the channel

WO 2010/044932 A2 provides an electrical detector comprising a nanofluidic channel and a charge sensor. A portion of the charge sensor is integrated in the nanofluidic channel whereby the charge sensor is contacted by fluid in the nanofluidic channel.

WO 2013/154999 A2 provides systems and methods for sequencing nucleic acids using nucleotide analogues and translocation of tags from incorporated nucleotide analogues through a nanopore.

EP 2652153 A2 provides a method of sequencing a single-stranded DNA using deoxynucleotide polyphosphate analogues and translocation of tags from incorporated deoxynucleotide polyphosphate analogues through a nanopore

WO 0070073 A1 provides a method of sequencing a target nucleic acid molecule having a plurality of nucleotide bases comprising: providing a complex of a nucleic acid polymerizing enzyme and the target nucleic acid molecule oriented with respect to each other in a position suitable to add a nucleotide analog at an active site complementary to the target nucleic acid; providing a plurality of types of nucleotide analogs proximate to the active site, wherein each type of nucleotide analog is complementary to a different nucleotide in the target nucleic acid sequence; polymerizing a nucleotide analog at an active site, wherein the nucleotide analog being added is complementary to the nucleotide of the target nucleic acid, leaving the added nucleotide analog ready for subsequent addition of nucleotide analogs; identifying the nucleotide analog added at the active site as a result of said polymerizing; and repeating said providing a plurality of types of nucleotide analogs, said polymerizing, and said identifying so that the sequence of the target nucleic acid is determined.

### SUMMARY

According to one aspect of the invention, provided is a microwell electrode, comprising: a substrate and an insulating layer on a surface of the substrate; one or more first electrodes; one or more second electrodes each arranged opposite to each first electrode, wherein a channel is defined by and located between each first electrode and the second electrode opposite thereto, and the channel has at least one end in communicaton with a chamber; and one or more guiding electrodes located in the chamber, wherein the first electrode, the second electrode and the guiding electrodes are located on a surface of the insulating layer away from the substrate.

In an embodiment, the guiding electrode can guide a charged substance into the channel and/or control the movement of a charged substance in the channel.

In an embodiment, the microwell electrode further comprises: a first supporting element for supporting the one or more first electrodes. Preferably, each of the first electrodes is located on a sidewall of the first supporting element.

In an embodiment, the microwell electrode comprises a plurality of first electrodes and a plurality of first supporting elements, each of the first electrodes is supported by a corresponding first supporting element.

In an embodiment, the microwell electrode comprises a plurality of second electrodes, the microwell electrode further comprising: a plurality of second supporting elements, each of the second electrodes is supported by a corresponding second supporting element. Preferably, each of the second electrodes is located on a sidewall of a second supporting element.

In an embodiment, the first electrode comprises a plurality of segment separated from each other.

In an embodiment, the second electrode comprises a plurality of segment separated from each other.

In an embodiment, the first electrode comprises a plurality of segments separated from each other, and the microwell electrode further comprises a plurality of first supporting elements, each segment of the first electrode is supported by a corresponding first supporting element.

In an embodiment, the second electrode comprises a plurality of segments separated from each other, and the microwell electrode further comprises a plurality of second supporting elements, each segment of the second electrode is supported by a corresponding second supporting element.

In an embodiment, the first supporting element is a conductive element. In such an embodiment, a voltage can be applied to the first electrode via the first supporting element.

In an embodiment, the first supporting element is a non-conductive element. In such an embodiment, preferably, the first supporting element is mainly used to support the first electrode, and preferably, a voltage can be applied to the first electrode via a wire embedded in the first supporting element.

In an embodiment, the second supporting element is a conductive element. In such an embodiment, a voltage can be applied to the second electrode via the second supporting element.

In an embodiment, the second supporting element is a non-conductive element. In such an embodiment, preferably, the second supporting element is mainly used to support the second electrode. And preferably, a voltage can be applied to the second electrode via a wire embedded in the second supporting element.

In an embodiment, the microwell electrode may further comprise a nanostructure capable of immobilizing an enzyme or a chemical substance to be detected. In an embodiment, the nanostructure is located on the bottom or a sidewall of the chamber, or on the bottom or a sidewall of the channel, or on the guiding electrodes.

In an embodiment, the nanostructure is formed by a material selected from a group consisting of a metal, a metal oxide, an inorganic polymer, an organic polymer, or any combination thereof.

In an embodiment, the channel has a width of 0.5-100nm, for example, 1nm, 2nm, 10nm, 50nm, 80nm, etc; and/or the channel has a length of 50nm-100µm, for example,100nm, 500nm, 5µm, 10µm, 30µm, etc; and/or the channel has a depth of 0-10µm, for example, 100nm, 300nm, 1µm, 2µm, 8µm, etc.

In an embodiment, the first electrode has a thickness of 1-1000nm, for example, 30nm, 50nm, 200nm, 600nm, 800nm, etc.

In an embodiment, the second electrode has a thickness of 1-1000nm, for example, 30nm, 50nm, 200nm, 600nm, 800nm, etc.

In an embodiment, the first electrode and the second electrode are formed by the same material.

In an embodiment, the first electrode and the second electrode are formed by different materials.

In an embodiment, the first supporting element, the first electrode and the second electrode may be formed by the same material, or by different materials.

In an embodiment, the first electrode is formed by a material selected from a group consisting of platinum, gold, indium tin oxide, carbon-based material(s), silicon or other conductive materials; and/or the second electrode is formed by a material selected from a group consisting of platinum, gold, indium tin oxide, carbon-based material(s), silicon or other conductive materials; and/or the guiding electrodes are formed by a material selected from a group consisting of silicon, platinum, gold, indium tin oxide, or carbon-based material(s).

In an embodiment, the conductive element is formed by a material selected from a group consisting of silicon, platinum, gold, silver, indium tin oxide, carbon-based material(s) or other conductive materials.

In an embodiment, the non-conductive element is formed by a material selected from a group consisting of silicon oxide, silicon nitride, silicon oxynitride, borophosphosilicate glass and the like.

In an embodiment, the first supporting element has a sectional shape of elliptical, circular, polygonal, or gear shape in a direction parallel to a surface of a substrate.

In an embodiment, the bottom surface of the chamber and the bottom surface of the channel are in the same plane or different planes.

In an embodiment, the microwell electrode further comprises: a substrate and an insulating layer on the substrate, where the first electrode, the second electrode and the guiding electrodes are located on the insulating layer.

In an embodiment, the guiding electrodes are substantially perpendicular to the first electrode or the second electrode. That is, the electric field direction between the guiding electrodes is substantially perpendicular to the electric field direction between the first electrode and the second electrode after applying voltages to the guiding electrodes, the first electrode and the second electrode.

In an embodiment, the microwell electrode further comprises: a passivation layer located on the surface(s) of the first electrode and/or the second electrode.

According to another aspect of the invention, provided is a microwell electrode array, comprising: one or more microwell electrodes according to any one of the above embodiments.

In an embodiment, the microwell electrode array comprises a plurality of microwell electrodes. For example, the number of the microwell electrodes may be 100, 10000, 10⁶ or 10⁸, etc.

In an embodiment, the plurality of microwell electrodes in the microwell electrode array are arranged in an elliptical, a circular, an annular, a fan, a rectangular, a square, a zigzag, or a gear shape, or as a matrix of rows and columns, or as laminated layers, etc.

In an embodiment, more than one microwell electrodes are independent from each other, or connected in series, or connected in parallel.

In an embodiment, the plurality of microwell electrodes in the microwell electrode array share one guiding electrode.

According to another aspect of the invention, a sensor chip is provided, comprising: the microwell electrode array according to the above embodiment.

In the invention, the sensor chip and a corresponding integrated circuit can be manufactured in a process matched with the CMOS process. In specific applications, the number of microwell electrodes included in a microwell electrode array on a sensor chip can be determined according to the size of the microwell electrode, the properties of a molecule to be detected, cost or other factors. For example, the microwell electrode array may be, as an exemple, a 10×10 array, 100×100 array, 1000×1000 array or 10⁴×10⁴ array, etc.

According to still another aspect of the invention, provided is a sequencing system, comprising: the sensor chip according to the above embodiment.

According to yet another aspect of the invention, provided is a method for manufacturing a microwell electrode, comprising: providing a substrate structure comprising a substrate with an insulating layer on its surface and a first supporting element material layer on the insulating layer, wherein the first supporting element material layer has successively on its sidewall a first electrode material layer, a sacrificial material layer, a second electrode material layer and a second supporting element material layer; patterning the first supporting element material layer, the first electrode material layer, the sacrificial material layer, the second electrode material layer and the second supporting element material layer to form one or more chambers, a first supporting element, and form a first electrode, a sacrificial layer, a second electrode and a second supporting element successively located on the sidewall of the first supporting element; forming one or more guiding electrodes in the chamber; removing the sacrificial layer on the sidewall of the first supporting element to form a channel between the first electrode and the second electrode, wherein the channel has at least one end in communication with the chamber.

In an embodiment, the step of providing the substrate structure comprises: providing a substrate with an insulating layer on its surface; forming a first supporting element material layer on a portion of the insulating layer; depositing a first electrode material layer to cover the upper surface and a sidewall of the first supporting element material layer; removing the first electrode material layer on the upper surface of the first supporting element material layer; depositing a sacrificial material layer to cover the upper surface of the first supporting element material layer, the upper surface and a sidewall of the remaining first electrode material layer; removing the sacrificial material layer on the upper surface of the first supporting element material layer and the upper surface of the remaining first electrode material layer; depositing a second electrode material layer to cover the upper surface of the first supporting element material layer, the upper surface of the remaining first electrode material layer and a upper surface and a sidewall of the remaining sacrificial material layer; removing the second electrode material layer on the upper surface of the first supporting element material layer, the upper surface of the remaining first electrode material layer, and the upper surface of the remaining sacrificial material layer; depositing a second supporting element material layer to cover the first supporting element material layer, the first electrode material layer on the sidewall of the first supporting element material layer, the sacrificial material layer over the sidewall of the first supporting element material layer, the second electrode material layer over the sidewall of the first supporting element material layer, and the portion which is not covered of the insulating layer; planarizing the deposited second supporting element material layer to expose the sacrificial material layer over the sidewall of the first supporting element material layer.

In an embodiment, the guiding electrodes are substantially perpendicular to the first electrode; and/or the guiding electrodes are substantially perpendicular to the second electrode.

In an embodiment, before removing the sacrificial layer on the sidewall of the first supporting element, the method further comprises: forming a passivation layer on a surface of at least one of the first supporting element, the second supporting element, the first electrode, or the second electrode.

In an embodiment, before removing the sacrificial layer on the sidewall of the first supporting element, the method further comprises: removing a portion of the top of the first supporting element and a portion of the top of the second supporting element to expose a portion of the first electrode, a portion of the sacrificial layer and a portion of the second electrode; depositing a passivation layer on the remaining first supporting element, the remaining second supporting element, the exposed portion of the first electrode, the exposed portion of the sacrificial layer and the exposed portion of the second electrode; planarizing the deposited passivation layer to form a passivation layer on the remaining portion of the first supporting element and the remaining portion of the second supporting element, and expose the sacrificial layer.

In an embodiment, the step of patterning the first supporting element material layer, the first electrode material layer, the sacrificial material layer, the second electrode material layer and the second supporting element material layer comprises: separating the first electrode material layer and/or the second electrode material layer into a plurality of segments, so that the first electrode and/or the second electrode formed each comprises a plurality of segments separated from each other.

In an embodiment, the method further comprises: forming a nanostructure capable of immobilizing an enzyme or a chemical substance to be detected on the bottom or a sidewall of the chamber, or on the bottom or a sidewall of the channel, or on the guiding electrodes.

In an embodiment, the nanostructure is formed by a material selected from a group consisting of a metal, a metal oxide, an inorganic polymer, an organic polymer, or any combination thereof.

In an embodiment, the channel has a width of 0.5-100nm, for example, 1nm, 2nm, 10nm, 50nm, 80nm, etc; and/or the channel has a length of 50nm-100µm, for example,100nm, 500nm, 5µm, 10µm, 30µm, etc; and/or the channel has a depth of 0-10µm, for example, 100nm, 300nm, 1µm, 2µm, 8µm, etc.

In an embodiment, the first electrode has a thickness of 1-1000nm, for example, 30nm, 50nm, 200nm, 600nm, 800nm, etc.

In an embodiment, the first electrode and the second electrode are formed by the same material.

In an embodiment, the first electrode and the second electrode are formed by different materials.

In an embodiment, the first electrode is formed by a material selected from a group consisting of silicon, platinum, gold, indium tin oxide, or carbon-based material(s); and/or the sacrificial layer is formed by a material selected from a group consisting of chromium, tungsten, aluminum, aluminum oxide, silicon, silicon oxide, silicon nitride; and/or the second electrode is formed by a material selected from a group consisting of silicon, platinum, gold, silver, indium tin oxide, or carbon-based material(s); and/or the guiding electrodes are formed by a material selected from a group consisting of silicon, platinum, gold, silver, indium tin oxide, or carbon-based material(s).

In an embodiment, the first supporting element and the second supporting element comprise conductive elements.

In an embodiment, the conductive element is formed by a material selected from a group consisting of silicon, platinum, gold, silver, indium tin oxide, or carbon-based material(s).

In an embodiment, the first supporting element and/or the second supporting element have a sectional shape of elliptical, circular, rectangular, square, or gear shape in a direction parallel to a surface of the substrate.

The invention also provides a method for analysis of a chemical substance, comprising the steps of:
(1) providing a microwell electrode or a microwell electrode array according to the invention;
(2) adding a reaction solution containing a chemical substance to be tested to the microwell electrode or microwell electrode array, and subjecting the reaction solution to a reaction to produce a charged molecule;
(3) allowing the charged molecule to enter the channel under the action of the guiding electrode and/or a hydromechanics effect, or to be accumulated in the channel under the action of the guiding electrode; and
(4) identifying the type of the charged molecule by using the first electrode, the second electrode and/or the guiding electrode, and therefore obtaining the information of the chemical substance to be tested.

In an embodiment, in the step (4), the type of the charged molecule is identified with the first electrode, the second electrode and/or the guiding electrode based on one or more effects selected from a group consisting of oxidation-reduction effect, electric resistance effect, capacitance effect, field effect, and tunneling effect.

In an embodiment, the chemical substance to be tested is selected from a group consisting of a biological molecule (such as a nucleic acid, protein, lipid, and polysaccharide), a compound, an organic polymer, etc. In an embodiment, the chemical substance to be tested is a nucleic acid, such as DNA or RNA.

In an embodiment, the chemical substance to be tested comprises or consists of one or more basic units (such as nucleotides, amino acids, and polymeric monomers). In an embodiment, the basic unit of the chemical substance to be tested is unmodified. In another embodiment, the basic unit of the chemical substance to be tested is modified by a label molecule.

In an embodiment, the reaction solution contains a free basic unit modified by a label molecule, which release a free label molecule after the reaction. Preferably, the free label molecule is charged (i.e., a charged molecule), and can enter the channel under the action of the guiding electrode, or be accumulated in the channel under the action of the guiding electrode. Therefore, the type of the charged molecule can be identified by using the first electrode, the second electrode and/or the guiding electrode, and therefore the information of the chemical substance to be tested is obtained.

In an embodiment, the reaction solution contains one or more types of free, label molecule-modified basic units. In an embodiment, the reaction solution contains at least two types (such as three, four, or more types) of free, label molecule-modified basic units. In an embodiment, different basic units are modified by the same label molecule. In another embodiment, different basic units are modified by different label molecules.

In an embodiment, the free label molecule is a redox active substance, and the first electrode and the second electrode are used as detection electrodes, to identify the type of the label molecule by an oxidation-reduction effect.

In an embodiment, in addition to the oxidation-reduction effect, in the step (4), the type of the charged molecule can be identified with the first electrode, the second electrode and/or the guiding electrode based on one or more electric resistance effect, capacitance effect, field effect, and tunneling effect. In such embodiments, the charged molecule can be detected by using one or more detection principles, so as to improve the accuracy of the detection result. For example, when an electric field is generated between guiding electrodes, any molecule in a channel will physically block ion current, thereby resulting in a detectable reduction in ion current. The accuracy of the detection of the charged molecule can be further improved by selecting a label molecule of a suitable size and a channel of a suitable length, and by the signal characteristic resulted from the electric resistance effect. Therefore, a person skilled in the art can select a combination of methods for identification of the types of the charged molecule depending on practical need. For example, a combination of field effect and oxidation-reduction effect, a combination of capacitance effect and oxidation-reduction effect, a combination of electric resistance effect and oxidation-reduction effect, or a combination of oxidation-reduction effect, electric resistance effect and field effect, can be used to identify the types of the charged molecule.

In an embodiment, there may be a modification film on the surface of an insulated layer on the bottom of the channel. Preferably, the first electrode and the second electrode are used as a source electrode and a drain electrode, respectively, and the modification film is used as a conducting channel between the two electrodes. Further preferably, a recognition site for a label molecule can be formed on the conducting channel and used as a gate electrode, so as to form a field-effect transistor. When different label molecules are attached on the modification film, the conducting channel will have different electric conductivity, thereby generating different current strength between the source electrode and the drain electrode. Therefore, based on the difference in current strength, different label molecules can be distinguished.

With respect to the reliability of a system, since the detection means do not overlap or affect each other, their results can be used as backup for each other, and use of multiple detection means will not result in collapse of the whole system.

In an embodiment, the first electrode and/or the second electrode each can be separated into a plurality of segments, i.e., two or more segments (e.g., 3-4 segments). In an embodiment, after the separation, each segment of electrode has a different voltage. In such embodiments, particularly preferably, the voltage of each segment of electrode corresponds to the response voltage (redox potential window) of a label molecule. Therefore, for a label molecule, only the segment of electrode, the voltage of which corresponds to the response voltage of the label molecule, can respond to the label molecule and generate a signal; while the other segments of electrode can neither respond to the label molecule nor generate a signal. Therefore, based on the presence or absence of a signal of a segment of electrode, it can be determined as to whether a label molecule corresponding to the segment of electrode is present or not. Such a detection means is favorable for distinguishing a signal from noise. Such a design on the electrodes is capable of providing dynamic information and is significantly different from the traditional nanopore-based method. In an embodiment, in the same channel, the first electrode and/or the second electrode is separated into a plurality of segments, such as 2-4 segments of transverse electrodes, and each single segment of electrode can be controlled independently so as to generate a different voltage.

In addition, in an embodiment, each microwell electrode may have a plurality of channels. A plurality of channels provided in a microwell electrode can greatly increase surface area for detection, and therefore can not only improve signal strength, but also reduce potential contamination effect. The combination of a microwell electrode array and a chamber also provides more controllable modes for sample injection.

In an embodiment, the channel may be open-ended. The process for manufacturing an open-ended channel is more simple and easier, and the open-ended channel is convenient for the injection of a sample and a liquid, and can better achieve a balance between speed and accuracy. In an embodiment, the channel may be close-ended. The close-ended channel is favorable for controlling and reducing the interference of external impurity signal. In practical application, the structure of the channels (open-ended channel, close-ended channel or a combination thereof) can be selected depending on particular conditions. In an embodiment, the microwell electrode comprises open-ended channel(s), or close-ended channel(s), or both of open-ended channel(s) and close-ended channel(s).

In an embodiment, the chemical substance to be tested is a nucleic acid molecule, and in the step (2), the reaction solution is subjected to polymerization of nucleotides.

In an embodiment, the method is used for analysis of the composition, sequence, electric charge, size or concentration of a chemical substance.

The invention also provides a method for analysis of a nucleic acid molecule, comprising the steps of:
(1) providing a microwell electrode or a microwell electrode array according to the invention;
(2) immobilizing a polymerase (such as DNA polymerase or RNA polymerase) in the chamber or channel of or on the guiding electrode of the microwell electrode or microwell electrode array;
(3) adding to the microwell electrode or microwell electrode array, a reaction solution containing a nucleic acid molecule to be tested, a primer, and at least one (e.g., one, two, three, or four) deoxyribonucleoside triphosphate (dNTP) molecule, nucleoside triphosphate (NTP) molecule or an analogue thereof, wherein the primer can hybridize or anneal to a partial sequence of the nucleic acid molecule to be tested, and each of the at least one dNTP or NTP molecule or analogue is modified with a label molecule, respectively; and later, under a suitable condition, hybridizing the nucleic acid molecule to be tested with the primer to form a complex;
(4) in the presence of the polymerase as a catalyst, incorporating one of the label molecule-modified dNTP or NTP molecule or analogue into the primer, to form an extension product complementary to the nucleic acid molecule to be tested, and removing the label molecule carried by the dNTP or NTP molecule or analogue incorporated into the primer, to provide a free label molecule, wherein the free label molecule is charged;
(5) allowing the free label molecule to enter the channel under the action of the guiding electrode and/or a hydromechanics effect, or to be accumulated in the channel under the action of the guiding electrode; preferably, the free label molecule is controlled to enter or accumulate into different microwell electrode channels by its electrical polarity or release order;
(6) identifying the type of the free label molecule by using the first electrode and the second electrode; and further identifying the type of the dNTP or NTP molecule or analogue incorporated into the primer according to the correspondence between the label molecule and the dNTP or NTP molecule or analogue; and further determining the base at the corresponding position of the nucleic acid molecule to be tested, according to the principle of complementary base pairing; and
(7) repeating the steps (4), (5) and (6) until the extension of the complex is finished.

In an embodiment, in the step (6), the type of the free label molecule is identified by one or more of oxidation-reduction effect, electric resistance effect, capacitance effect, field effect, and tunneling effect.

In an embodiment, the type of the label molecule is identified by oxidation-reduction effect. In an embodiment, the free label molecule may be a redox active substance that is reactive in a circular redox reaction, or may be converted to a redox active substance that is reactive in a circular redox reaction. In an embodiment, the free label molecule may be physically or chemically converted to a redox active substance that is reactive in a circular redox reaction. Preferably, the redox active substance can be subjected to a circular redox reaction between the first electrode and the second electrode, resulting in a detectable current. Thereby, the type of the label molecule can be identified by the detectable current. In an embodiment, in addition to the above-mentioned oxidization-reduction effect, the type of the label molecule can be identified by one or more of electric resistance effect, capacitance effect, field effect, and tunneling effect.

In an embodiment, the entrance of the free label molecule (e.g., a redox active label molecule) is led and/or controlled by using the guiding electrode or other means, and potentials are applied to the first electrode and the second electrode of the channel, respectively. When the potential of the first electrode and the second electrode matches the redox potential window of the label molecule, the label molecule can be subjected to a circular redox reaction between the first electrode and the second electrode, and a detectable redox current pulse signal is generated. By using the detectable redox current pulse signal, the label molecule can be specifically identified and detected. When a matching potential is provided between the first electrode and the second electrode but no pulse signal is detected, it indicates that the label molecule is not present.

In an embodiment, each of the at least one dNTP or NTP molecule or analogue carries a label molecule having a different redox potential window. In a preferred embodiment, the type of the label molecule in the channel can be identified by changing the potential of the first electrode and/or the second electrode, and determining whether a redox current pulse signal is generated under various potential conditions, and optionally, determining the information such as the signal amplitude of the pulse signal.

In an embodiment, each of the at least one dNTP or NTP molecule or analogue carries a label molecule having a different redox potential window. In a preferred embodiment, the first electrode and/or the second electrode in the channel is separated into a plurality of segments, and each segment is applied with a potential corresponding to the redox potential window of a different label molecule. Therefore, when a label molecule passes the channel, a redox current pulse signal can only be detected in the electrode segment with a potential corresponding to the redox potential window of the label molecule, thereby identifying the type of the label molecule in the channel.

In an embodiment, the reaction solution further comprises a phosphatase.

In an embodiment, in the step (4) of the method, the free label molecule is dephosphorylated in the presence of a phosphatase.

In an embodiment, the label molecule-modified dNTP, NTP or analogue is neutral in net charge or is negatively charged.

In an embodiment, the free label molecule is positively charged or negatively charged.

In an embodiment, the label molecule-modified dNTP, NTP or analogue is neutral in net charge or is negatively charged, and the free label molecule is positively charged. In such embodiments, the positively charged free label molecule can migrate along the channel under the action of an electric field, while the label molecule-modified dNTP, NTP or analogue will not migrate in the chamber or channel as they are neutral or negatively charged.

In an embodiment, the label molecule-modified dNTP, NTP or analogue is negatively charged, and the free label molecule is negatively charged. In such embodiments, the free label molecule, the label molecule-modified dNTP, NTP or analogue, and the unmodified dNTP, NTP molecule, all of which are negatively charged, can migrate together along the channel under the action of an electric field.

In an embodiment, only the free label molecule is redox active, while the label molecule-modified dNTP, NTP molecule and the unmodified dNTP, NTP molecule are not redox active. In such embodiments, the redox current signal can only be resulted from the free label molecule.

In an embodiment, the label molecule is linked to the phosphate group, base or saccharide group of the dNTP or NTP molecule or analogue. Preferably, the label molecule is selected from one or more of: amino acids, peptides, carbohydrates, metal compounds, dyes, chemiluminescent compounds, nucleotides, aliphatic acids, aromatic acids, alcohol, aminophenyl, hydroxyphenyl, naphthyl, thiol group, cyano, nitro, alkyl, alkenyl, alkynyl, azido, or a derivative thereof. Preferably, the label molecule is selected from one or more of: aminophenyl, hydroxyphenyl, naphthyl, variable-valency metal compounds (such as ferrocene, hexacyanoferrate, and ferrocyanide), anthraquinone, and methylene blue, and a derivative thereof. In an embodiment, the label molecule is linked to the γ-phosphate group of the dNTP or NTP molecule or analogue, and preferably, the label molecule is selected from a group consisting of aminophenyl, hydroxyphenyl, naphthyl, and a derivative thereof. In an embodiment, the label molecule is linked to the base or saccharide group of the dNTP or NTP molecule or analogue; and preferably, the label molecule is selected from a group consisting of variable-valency metal compounds (such as ferrocene, hexacyanoferrate, and ferrocyanide), anthraquinone, and methylene blue, and a derivative thereof.

In an embodiment, each type of dNTP (e.g., dATP, dTTP, dCTP, dGTP, dUTP) or NTP (e.g., ATP, TTP, CTP, GTP, UTP) molecule is labelled with a specific label molecule having a redox activity, wherein the specific label molecule can generate a specific redox electric signal when subjected to a circular redox reaction.

In an embodiment, in the step (3), all the four types of dNTP (for example, selected from a group consisting of dATP, dTTP/dUTP, dCTP, dGTP) or NTP (e.g., ATP, TTP, CTP, GTP, UTP) molecules are added simultaneously. In such embodiments, the washing step following the incorporation of each base may be omitted, thereby greatly reducing the cost of reagents and accelerating the detection speed.

In an embodiment, the charge carried by the free label molecule can be adjusted by selecting a label molecule, so as to adjust the migration speed of the free label molecule under the action of a guiding electrode.

In an embodiment, in the step (1), the polymerase is immobilized on an insulated layer on the bottom of the chamber or channel, or immobilized on a guiding electrode. Preferably, the polymerase is immobilized at a place close to the inlet port of the channel at the bottom of the chamber; preferably, the inlet port of the channel may be designed in a variety of shapes (such as a funnel shape), to hold a polymerase.

In an embodiment, a polymerase is immobilized in each chamber or channel.

In an embodiment, the nucleic acid molecule to be tested in the reaction solution is a single-stranded nucleic acid molecule.

In an embodiment, each polymerase can capture a single-stranded nucleic acid molecule or a complex formed from the hybridization of a nucleic acid molecule and a primer.

In the invention, methods for immobilizing a polymerase on the bottom of a chamber or channel are well known in the art.

In an embodiment, the insulated layer is formed by a material selected from a group consisting of silicon dioxide, silicon oxynitride, silicon nitride or other insulating materials (e.g., Carbon Doped Oxide (CDO), silicon carbide, organic polymer such as polyimide, octafluorocyclobutane or polytetrafluoroethylene, fluorosilicate glass (FSG), and organic silicate such as silsesquioxane, siloxane or organic silicate glass).

In an embodiment, a functionalizable region and/or a molecule-binding region may be further provided between the insulated layer and the polymerase. In an embodiment, the functionalizable region comprises a functionalizable material such as silicon dioxide, hafnium oxide, aluminum oxide, tantalum oxide, and/or zirconium oxide. For example, the functionalizable material may be functionalized with a linking molecule selected from a group consisting of: silicane (e.g., aminopropyltriethoxysilane), thiol (-SH), disulfide (-S-S-), isothiocyanate, alkene and alkyne. In an embodiment, the molecule-binding region comprises a probe molecule. Preferably, the probe molecule is, for example, selected from a group consisting of a biotin, an avidin, an antibody, an antigen, a receptor, a ligand, a DNA sequence, a RNA sequence, a protein and a ligand thereof.

In an embodiment, a binding molecule can be selected so that the polymerase is immobilized in a suitable direction.

In an embodiment, the method is used for analysis of the sequence, composition, electric charge, size or concentration of a nucleic acid molecule.

In the invention, an electrolytic solution and a reaction solution may be added to the surface of the microwell electrode so that all the chambers and channels are filled with the electrolytic solution and the reaction solution. For different electrodes or different segments of the same electrode, their potentials can be set independently so as to control or detect reactive molecules independently.

In a redox detection mode, the guiding electrode controls the entrance of an electrolytic solution and a redox active substance into a channel, and the first electrode and the second electrode are used as a redox reaction detection device for detecting the redox active substance.

In the invention, the electric field formed by the guiding electrode is favorable for entrance of charged molecules (such as positively charged molecules) into a channel, and accumulation thereof in the channel, so as to reduce the possibility that the molecules diffuse out from the channel, which in turn can enhance the strength of the signal detected.

In order to ensure resolution of a single nucleotide, the voltage of the guiding electrode can be adjusted so as to control the transport speed of the charged molecules. In addition, the polymerase may also be modified so that the optimal synthesis speed matches the transport speed of the molecules. In addition, micro or nano-scale fluid mechanics may also be used to control the migration of molecules in a channel.

In the invention, the nucleic acid molecule includes deoxyribonucleic acid (DNA), ribonucleic acid (RNA), and polymers of other analogues linked together via phosphodiester bond. Polynucleotide may be a fragment of a genome, a gene or a part thereof, cDNA or a synthesized deoxyribonucleic acid sequence. Nucleotides included in a polynucleotide may be naturally occurring deoxyribonucleotides such as adenine, cytosine, guanine or thymine linked to 2'-deoxyribose, or ribonucleotides such as adenine, cytosine, guanine or uracil linked to ribose. However, a polynucleotide or oligonucleotide (such as a probe or primer) may further comprise a nucleotide analogue, including a non-naturally occurring synthetic nucleotide or a modified naturally occurring nucleotide.

In the invention, the redox cycle refers to an electrochemical method in which a molecule that can be reversely oxidized and/or reduced (i.e., a redox active molecule) migrates between at least two independently biased electrodes, wherein one of the at least two electrodes has a potential lower than the reduction potential of the redox active molecule to be detected, while the other of the at least two electrodes has a potential higher than the oxidation potential of the redox active molecule, so that electrons shuttle between the independently biased electrodes (i.e., the molecule is oxidized at the first electrode, and then diffuses to the second electrode and reduced there, or vice versa, the molecule is reduced and then oxidized, depending on the molecule and the potential of the electrode when biased). In a redox cycle, the same molecule can therefore contribute a plurality of electrons to the recorded current, resulting in net amplification of a signal. The signal resulted from a redox active substance can be potentially amplified by more than 100 folds, depending on the factors such as the stability of the redox active substance and the ability of the redox active substance to diffuse to a detectable region. In the invention, a guiding electrode can be provided to prevent the diffusion of a redox active substance outside the channel, so as to increase the effective concentration of the redox active substance in the channel.

In the invention, the redox active substance (or the redox active molecule) has the general meanings in the art, and is a molecule that can be reversely oxidized and/or reduced for many times.

In the invention, the phosphatase is, for example, selected from a group consisting of alkaline phosphatase, acid phosphatase, protein phosphatase, polyphosphatase, sugar-phosphatase and pyrophosphatase.

In the invention, during the synthesis of an extension product, the incorporation of the label molecule-modified dNTP, NTP or analogue releases the label molecule-pyrophosphate (PPi) into the solution. The phosphatase functions to remove pyrophosphate from the label molecule-pyrophosphate. The removal of the phosphate group further activates the redox active substance, and therefore the presence of the redox active substance can be detected by a electrochemical mean.

In the invention, the silane molecule can have the chemical formulae X₃-Si-YR", X2-Si-(N)YR" or X-Si-(N₂)YR", wherein X is a leaving group, such as -Cl, -OCH₃ or -OCH₂CH₃, R" is a reactive coupling group, such as -NH₂, -COOH, -COH, -CHCH₂ or -SH, and Y is a non-reactive group, such as an alkyl group. The organic group for use in coupling, as presented by the silane molecule attached on a surface, may be, for example, carboxyl group, aldehyde, ester, alkene, alkyne, thiol, isothiocyanate, isocyanate, substituted amine, epoxide, small molecule such as biotin, or ethanol. In general, Y is a non-reactive group, such as, a hydrocarbon compound having 1 to 16 carbon atoms. Examples of -YR" include -(CH₂)₃NH₂, -(CH₂)₂COOH and -(CH₂)₂SH. Some examplary silanes include (3-aminopropyl)triethoxysilane (APTS), mercaptosilane and glycidoxy trimethoxy silane (having the coupling group of an epoxide). The surface to be silylated may react with, for example, silane molecules in solution, or silane molecules as silane gas.

In the invention, the base, for example, is selected from a group consisting of adenine, guanine, cytosine, thymine, uracil, 7-deazaguanine, 7-deazaadenine, and 5-methylcytosine.

In the invention, the primer (primer sequence) is a short oligonucleotide of a suitable length (for example, about 18-24 bases in length) that is generally synthesized chemically, and is sufficient to be hybridized to a target nucleic acid (for example, a single-stranded DNA) and allow addition of nucleotide residues thereto or synthesis of an oligonucleotide or a polynucleotide therefrom under suitable conditions as well known in the art. In an embodiment, the primer is a DNA primer, i.e., a primer consisting or mainly consisting of deoxyribonucleotide residues. The primer is designed to having a reverse complementary sequence of a region of a template/target nucleic acid (such as a single-stranded DNA) which is hybridizable to the primer. Nucleotide residues are added to the 3' end of a primer by formation of a phosphodiester bond so as to produce an extension product; or nucleotide residues are added to the 3' end of the extension product by formation of a phosphodiester bond so as to produce another extension product.

In the invention, incorporation of the dNTP, NTP or analogue into an oligonucleotide or a polynucleotide (such as a primer, an extension product, a complex formed from a primer and a nucleic acid molecule to be tested) refers to the formation of a phosphodiester bond between the 3' carbon atom of the nucleotide residue at the 3' end of the polynucleotide and the 5' carbon atom of the dNTP, NTP or analogue.

In the invention, the polymerase includes DNA polymerase, RNA polymerase, reverse transciptase, and the like, the functions or kinds of which are well known in the art. The DNA polymerase may, for example, have 3' to 5' exonuclease activity or not, including for example, *E. coli* DNA polymerase, Klenow fragment, Phusion DNA polymerase, 9°N DNA polymerase, KOD polymerase, Therminator DNA polymerase, Taq DNA polymerase, Vent DNA polymerase, and the like.

In an embodiment, by modification of a polymerase, an ideal base incorporation rate (e.g., 1-100 bases per second) is achieved in real-time sequencing.

In an embodiment, a polymerase is specifically modified to be more suitable for application. For example, a polymerase, which can be used to synthesize a polymer quickly, continuously and accurately, is selected or obtained from a clone bank of DNA polymerases, with a proviso that a single DNA polymerase molecule can achieve synthesis of 1-100 kb DNA without dissociation from the template strand. Preferably, the polymerase lacks exonuclease activity, and the error rate of base incorporation shall be as low as 10⁻⁵-10⁻⁶/incorporated base. The bias of the polymerase for each specific nucleotide can greatly enhance the sequencing specificity. A polymerase having different detectable bias for DNA, RNA and methylated base is favorable for sequencing. In addition to the mentioned physio-chemical and zymologic properties, other properties may include thermal stability, stable buffer system, working capability under accumulation of macromolecules, and tolerance to a lot of side products (pyrophosphate).

In an embodiment, suitable mutants, which have a polymerization rate matching the ability of the detection device, are screened from a clone bank of polymerases, so that the true base incorporation and the non-incorporation can be distinguished from each other to the largest extent. In such a single-molecule/real-time polymerase method, the polymerase is also required to enable incorporation of modified nucleotides. Therefore, it needs to select polymerase mutants and modified nucleotides, which match the ability of the detection device.

In an embodiment, in addition to selection of the most suitable polymerase mutant, a buffer may also be used to change the base incorporation rate (for example, the incorporation rate of a specific nucleotide may be decreased or increased by virtue of pH and ions). By determining the detention time of nucleotides in a polymerase pocket, a polymerase mutant can be selected for its higher or lower bias for a specific nucleotide. By enzyme kinetic measurement, it can reduce the probability of false positive incorporation, and can distinguish a true incorporation signal from a device noise.

In an embodiment, by analyzing and comparing the crystal structures of the binary complex and ternary complex of polymerase mutants and matching them to detectable incorporation events, the residues in a candidate, which are important to specificially targeting mutations, can be found, thereby promoting discovery of a new specific and stable interaction.

Another characteristic of the enzyme is that it can release the nucleic acid molecule at the end point, so as to enable the entrance of a new primer-carrying template DNA and the sequencing to begin. In an embodiment, in the presence of a sufficiently stable enzyme, a 1-10-100-1000 kb template can be sequenced in a channel.

### ADVANTAGEOUS EFFECTS OF THE PRESENT INVENTION

A microwell electrode of the present invention has the following advantages: being able to comprise a plurality of nanochannels in a microwell electrode , thus reducing the influence of contamination of electrode surface; being able to guide the flow of a reagent by an electric field and electroosmotic flow generated by a guiding electrode in a nano-channel; being easy to integrate with a microfluid to achieve the purpose of reagent interaction; being easy for large-scale manufacturing of electrode arrays; being compatible with a variety of signal detection mechanisms, including electronic signal detection (e.g., based on a redox cycle, FET (field effect transistor), electrochemical, electrical impedance); making physical amplification of a signal possible; allowing the integration with a monolithic CMOS; being able to control the movement of a nucleic acid molecule by an electric field; enabling single molecule detection of a chemical substance to be detected by detecting an electrical signal of a label molecule; greatly increasing read length; and being sensitive for detection signal.

Method for analysis of a chemical substance of the present invention can be used in molecular detection and analysis, molecular diagnosis, disease detection, substance identification, and DNA detection and sequencing, etc.

Other features, aspects and advantages of the present invention will become apparent by reference to the following detailed description of exemplary embodiments of the present invention with reference to the drawings.

### DESCRIPTION OF THE DRAWINGS

The drawings, which constitute part of this specification, illustrate exemplary embodiments of the invention and, together with this specification, serve to explain the principles of the invention. In the drawings:
Fig. 1A is a top view of a microwell electrode according to an embodiment of the present invention;
Fig. 1B is a sectional view taken along B-B' shown in Fig. 1A;
Fig. 1C is a sectional view taken along C-C' shown in Fig. 1A;
Fig. 2A is a schematic diagram showing working principle of a microwell electrode according to an embodiment of the present invention;
Fig. 2B is a schematic diagram showing the corresponding relationship between different bases and detection voltages applied on a first electrode and a second electrode;
Fig. 3 is a schematic diagram showing a microwell electrode according to an embodiment of the present invention, wherein the first electrode and the second electrode each includes four segments;
Fig. 4A is a schematic diagram showing a microwell electrode array consisting of two microwell electrodes;
Fig. 4B is a schematic diagram showing a microwell electrode array consisting of four microwell electrodes;
Figs. 5A, 5B, 5C and 5D show four arrangements of a plurality of microwell electrodes in a microwell electrode array, respectively;
Fig. 6 is a schematic diagram showing a sequencing system according to an embodiment of the present invention;
Fig. 7 is a schematic diagram showing a simplified flow of a method for manufacturing a microwell electrode according to an embodiment of the present invention;
Fig. 8A shows a sectional view of a substrate structure according to an embodiment of the present invention;
Fig. 8B shows a top view of the substrate structure shown in Fig. 8A;
Fig. 8C is a top view of a product at a stage of a method for manufacturing a microwell electrode according to an embodiment of the present invention;
Fig. 8D is a top view of a product at a stage of a method for manufacturing a microwell electrode according to an embodiment of the present invention;
Fig. 8E is a sectional view of a product at a stage of a method for manufacturing a microwell electrode according to an embodiment of the present invention;
Fig. 8F is a sectional view of a product at a stage of a method for manufacturing a microwell electrode according to an embodiment of the present invention;
Fig. 8G is a sectional view of a product at a stage of a method for manufacturing a microwell electrode according to an embodiment of the present invention;
Fig. 8H is a sectional view of a product at a stage of a method for manufacturing a microwell electrode according to an embodiment of the present invention;
Fig. 8I is a sectional view of a product at a stage of a method for manufacturing a microwell electrode according to an embodiment of the present invention;
Fig. 8J is a sectional view of a product at a stage of a method for manufacturing a microwell electrode according to an embodiment of the present invention;
Fig. 8K is a sectional view of a product at a stage of a method for manufacturing a microwell electrode according to an embodiment of the present invention;
Fig. 9A is a sectional view of a product at a stage of forming a substrate structure according to an embodiment of the present invention;
Fig. 9B is a sectional view of a product at a stage of forming a substrate structure according to an embodiment of the present invention;
Fig. 9C is a sectional view of a product at a stage of forming a substrate structure according to an embodiment of the present invention;
Fig. 9D is a sectional view of a product at a stage of forming a substrate structure according to an embodiment of the present invention;
Fig. 9E is a sectional view of a product at a stage of forming a substrate structure according to an embodiment of the present invention;
Fig. 9F is a sectional view of a product at a stage of forming a substrate structure according to an embodiment of the present invention;
Fig. 9G is a sectional view of a product at a stage of forming a substrate structure according to an embodiment of the present invention;
Fig. 9H is a sectional view of a product at a stage of forming a substrate structure according to an embodiment of the present invention;
Fig. 9I is a sectional view of a product at a stage of forming a substrate structure according to an embodiment of the present invention;
Fig. 9J is a sectional view of a product at a stage of forming a substrate structure according to an embodiment of the present invention;
Figs. 10A and 10B show the detection results of different free label molecules detected by using an exemplary microwell electrode of the present invention, respectively;
Fig. 11 gives distribution of the number of collisions between a single label molecule and an electrode in an exemplary microwell electrode of the present invention, which is obtained through a simulation calculation.

### DETAILED DESCRIPTION

Various exemplary embodiments of the present invention will now be described in detail with reference to the drawings. It should be understood that the relative arrangement, numerical expressions and numerical values of the components and steps set forth in these embodiments, unless otherwise specified, should not be constructed as limiting the scope of the invention. For specific conditions not specified in the embodiments, routine conditions or conditions recommended by manufacturers are generally used. Reagents or instruments without specified manufacturers are all commercially available products.

Besides, it should be understood that, for the convenience of description, the dimensions of the various components shown in the drawings are not necessarily drawn in accordance with actual proportions, for example, a thickness or a width of certain layers may be exaggerated relative to other layers.

The following description of exemplary embodiments is merely illustrative and is not intended to be construed as limiting the invention and its application or use in any way.

Techniques, methods, and apparatus known to those of ordinary skill in the relevant art may not be discussed in detail, but these techniques, methods, and apparatuses should be considered as part of the specification where appropriate.

It should be noted that similar reference numerals and letters are used to denote similar items in the following drawings, and therefore, once an item is defined in a drawing, it is not necessary to further discuss it in the illustration of subsequent drawings.

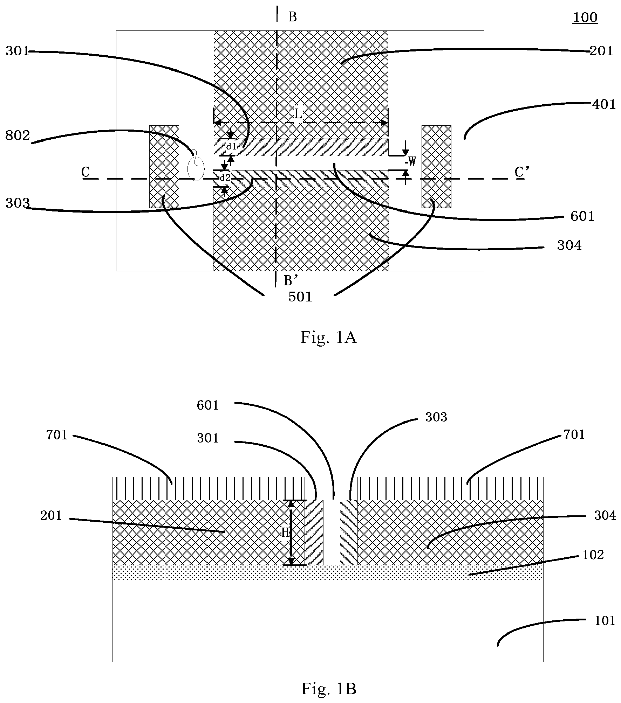

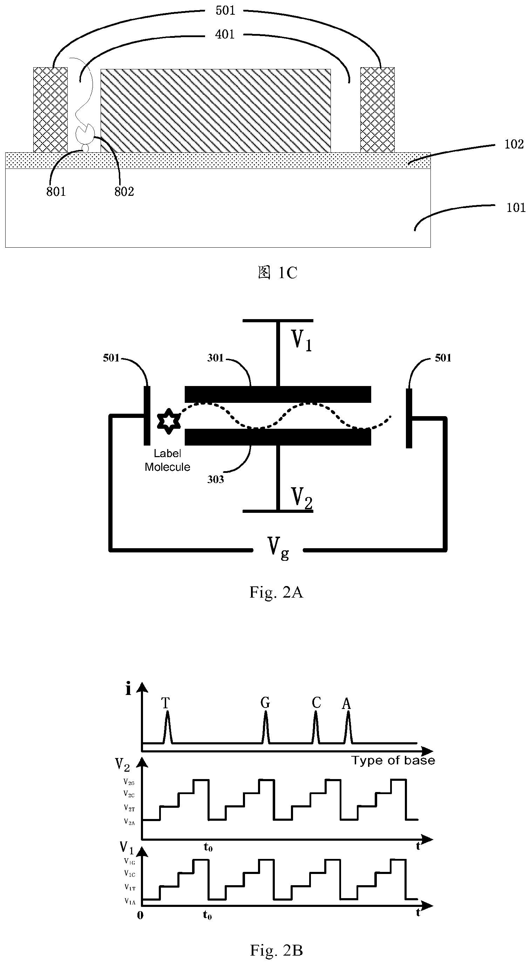

Fig. 1A is a top view of a microwell electrode according to an embodiment of the present invention. Fig. 1B is a sectional view taken along B-B' shown in Fig. 1A. Fig. 1C is a sectional view taken along C-C' shown in Fig. 1A.

Refering to Figs. 1A, 1B and 1C, a microwell electrode 100 may comprise one or more first electrode(s) 301. Different first electrodes 301 can be separated from each other, for example, different first electrodes 301 may have gaps therebetween or the gaps may be filled with an insulating layer. Exemplarily, the first electrode 301 may have a thickness d1 of about 1-1000nm, for example, 30nm, 50nm, 200nm, 600nm, 800nm or the like. In an embodiment, the first electrode 301 may be formed by a material selected from a group consisting of platinum, gold, silver, indium tin oxide, carbon-based materials (for example, diamond, graphite, amorphous carbon, carbon nanotubes, etc), silicon or any combination thereof.

The microwell electrode 100 further comprises one or more second electrodes 303 each arranged opposite to one first electrode 301. A channel 601 is provided between each first electrode 301 and its opposite second electrode 303. The channel 601 has at least one end in communication with a chamber 401. Exemplarily, the second electrode 303 may have a thickness d2 of about 1-1000nm, for example, 30nm, 50nm, 200nm, 600nm, 800nm or the like. In one embodiment, the second electrode 303 may be formed by a material selected from a group consisting of platinum, gold, silver, indium tin oxide, carbon-based materials (for example, diamond, graphite, amorphous carbon, carbon nanotubes, etc), silicon or any combination thereof. In an embodiment, the bottom surface of the chamber 401 and the bottom surface of the channel 601 may be in the same plane. In another embodiment, the bottom surface of the chamber 401 and the bottom surface of the channel 601 may be in different planes.

The microwell electrode 100 further comprises one or more guiding electrodes 501 located in the chamber 401. In an embodiment, the guiding electrode 501 may lead a charged substance into the channel 601. Further, the guiding electrode 501 may also control the movement of a charged substance in the channel 601. When different voltages are applied on the guiding electrodes 501 at both ends of the channel 601, a leading electric field produced between the guiding electrodes 501 can control thea charged substance (for example, a redox-active molecule) to enter into the channel 601, and control the movement of a charged substance (for example, a redox-active molecule) in the channel 601 so that the charged substance cannot easily move outside the channel 601. If only one guiding electrode 501 is provided in the chamber 401, this guiding electrode 501 can be used to control the movement of a charged substance in one or more channels 601 that communicate with the chamber 401. If a plurality of guiding electrodes 501 are provided in the chamber 401, each of the guiding electrodes 501 can control the movement of a charged substance in an adjacent channel 601 thereto, respectively. Exemplarily, the guiding electrode 501 may be formed by a material selected from a group consisting of silicon, platinum, gold, ITO, carbon-based materials or any combination thereof.

Preferably, the guiding electrode 501 can be substantially perpendicular to the first electrode 301 or the second electrode 303 (as shown in Fig. 1). However, it should be understood that the guiding electrode 501 is not necessarily perpendicular to the first electrode 301 or the second electrode 303, and an angle of other degree therebetween is also possible.

A microwell electrode provided in the present invention can be used in, but not limited to, nucleic acid sequencing, in which case, the chamber 401 may be a microfluidic chamber. The manufacturing process of a microwell electrode can be compatible with CMOS process, and is easy to mass manufacture. Further, a plurality of signaling mechanisms can be adopted in nucleic acid sequencing and a longer read length can be achieved. Further, since a microwell electrode may comprise multiple channels at the same time, the effect of electrode contamination on sequencing results can be reduced, thus making sequencing results more accurate.

Fig. 2A is a schematic diagram showing working principle of a microwell electrode according to an embodiment of the present invention. As shown in Fig. 2A, the microwell electrode comprises a set of detection electrodes (a first electrode 301 and a second electrode 303) and a set of guiding electrodes 501. A voltage V₁ is applied on the first electrode 301, a voltage V₂ is applied on the second electrode 303, and a voltage difference V_{g} is produced between the two guiding electrodes 501. A leading electric field between the two guiding electrodes 501 can pull a molecule to be detected (for example, a redox-active label molecule) into a channel between the two detection electrodes. The label molecule undergoes a redox cycle reaction between the two detection electrodes. The redox cycle reaction produces a corresponding current signal, which can be detected by the two detection electrodes. Different label molecules correspond to different current signals; therefore, the type of a label molecule can be determined according to the detected current signal.

Fig. 2B is a schematic diagram showing the corresponding relationship between different bases and detection voltages applied on a first electrode and a second electrode. According to the principle of redox reaction, only when the voltages V₁ and V₂ are in a redox potential window corresponding to a molecule to be detected, a current signal caused by the molecule to be detected can be detected. Thus, the voltages V₁ and V₂ can be put in a redox potential window corresponding to a molecule to be detected by changing the values of the voltages V₁ and V₂ several times in a time period to, and then the type of the molecule to be detected can be identified. In this way, current signals of different molecules to be detected can be detected at different voltages V₁ and V₂. As shown in Fig. 2B, bases A, T, C, and G correspond to different voltages V₁ and V₂, respectively. Specifically, base A corresponds to voltages V_{1A} and V_{2A}, base T corresponds to voltages V_{1T} and V_{2T}, base C corresponds to voltages V_{1C} and V_{2C}, and base G corresponds to voltages V_{1G} and V_{2G}. Taken base A as an example, when the voltage V₁ is set to V_{1A} and the voltage V₂ is set to V_{2A}, if a label molecule corresponding to base A is present in the channel, a current signal i produced by the label molecule corresponding to base A in a redox reaction can be detected by the detection electrodes. Similarly, by changing the values of the voltages V₁ and V₂, the voltages V₁ and V₂ can be put in the redox potential windows of the label molecules corresponding to bases T, C or G, the type of a label molecule can be identified, and then the type of the base corresponding to the label molecule can be identified.

In an embodiment, refering to Figs. 1B and 1C, the microwell electrode may further comprise a substrate 101 and an insulating layer 102 on the substrate 101. The first electrode 301, the second electrode 303 and the guiding electrodes 501 can be disposed on the insulating layer 102. The substrate 101 may be, for example, a silicon substrate, a substrate made of a Group III-V semiconductor material, a silicon on insulator (SOI) substrate, or may be an oxide semiconductor (such as ZnO, CdO, TiO₂, Al₂O₃, or SnO) substrate, or may be a substrate made of an insulating material, such as a quartz glass, or a soda glass. The insulating layer 102 may be typically a silicon oxide (e.g., silicon dioxide), a silicon nitride (e.g., SiN), a silicon nitrogen oxide, etc.

In practical applications, the above first electrode 301 and second electrode 303 can be supported by supporting elements. In an embodiment, refering to Fig. 1A, the microwell electrode may further comprise a first supporting element 201 for supporting the first electrode 301. In an embodiment, the microwell electrode may comprise a plurality of first electrodes 301, all of which can be supported by the first supporting element 201. In another embodiment, the microwell electrode may comprise a plurality of first electrodes 301 and a plurality of first supporting elements 201, and each of the first electrodes 301 can be supported by a corresponding first supporting element 201, respectively. In still another embodiment, the microwell electrode may comprise a plurality of second electrodes 303 and a plurality of second supporting elements 304, and each of the second electrodes 303 can be supported by a corresponding second supporting element 304, respectively.

The above first supporting element 201 and second supporting element 304 may be conductive elements or non-conductive elements, and preferably, conductive elements. In the case where a first electrode 201 and a second electrode 304 are non-conductive elements, the first supporting element 201 and the second supporting element 304 mainly serve to support the first electrode 301 and the second electrode 303. In the case where a first electrode 201 and a second electrode 304 are conductive elements, voltages can be applied to the first electrode 301 and the second electrode 303 through applying voltages to the first supporting element 201 and the second supporting element 304. In an embodiment, the above conductive elements may be formed by a material selected from a group consisting of silicon (for example, polysilicon), platinum, gold, silver, indium tin oxide, or carbon-based material(s), etc. The above non-conductive elements may be formed by a material selected from silicon oxide, silicon nitride, silicon oxynitride, borophosphosilicate glass, and the like. Further, the first supporting element 201 may have a sectional shape of an elliptical, circular, polygonal, square or gear shape in the direction parallel to the surface of the substrate 101. It should be understood that the present invention is not limited to the above exemplary shapes.

In another embodiment of a microwell electrode of the present invention, the material of the above first electrode 301 and the material of the second electrode 303 may be the same or may be different. In an embodiment, one of the first electrode 301 and the second electrode 303 may be made of an electrode material which is easy to be oxidized, and the other may be made of an electrode material liable to be reduced.

In another embodiment of a microwell electrode of the present invention, as shown in Fig. 1B, the microwell electrode 100 may further comprise a passivation layer 701 formed on the surface of at least one of the first electrode 301, the second electrode 303, the first supporting element 201 (if any), the second supporting element 304 (if any). In an embodiment, the passivation layer 701 may be a silicon nitride (e.g., SiN), a silicon oxide (e.g., SiO₂) and the like. The passivation layer 701 may serve to protect the microwell electrode. Specifically, when performing nucleic acid sequencing by the microwell electrode, only a region that is not covered by the passivation layer 701 can contact reaction solution, so that damages to certain components of the microwell electrode can be avoided. In addition, the passivation layer 701 may also reduce noises produced in the sequencing process.

In still another embodiment of a microwell electrode of the present invention, the microwell electrode 100 may further comprise a nanostructure 801 (e.g., a nanodot) capable of immobilizing an enzyme or a chemical substance 802 to be detected. The nanostructure 801 can be located on the bottom or a sidewall of the chamber 401 (as shown in Figs. 1A and 1B), or on the bottom or a sidewall of the channel 601 (not shown), or on the guiding electrode 211. Exemplarily, the size of the nanostructure 801 may be 1-100nm, for example, 8nm, 20nm, 60nm and the like. Preferably, the nanostructure 801 may be formed by a metal oxide; preferably, the metal oxide is a transition metal oxide such as zirconium dioxide (ZrO₂) or hafnium dioxide (HfO₂). Alternatively, the nanostructure 801 may also be formed by a metal; preferably, the metal is an inert metal such as gold or platinum. Alternatively, the nanostructure 801 may also be formed by a material selected from an inorganic polymer, an organic polymer, or any combination thereof.

In some implementations, the first electrode 301 and/or the second electrode 303 may comprise a plurality of segments separated from each other, for example, two or more segments. A description will be given below with reference to different implementations.

In one implementation, the first electrode 301 comprises a plurality of segments separated from each other, and the second electrode 303 comprises only one segment. The microwell electrode may comprise a plurality of first supporting elements 201, each segment of the first electrode 301 can be supported by a corresponding first supporting element 201. In this case, a voltage can be applied to the second electrode 303 and voltages corresponding to different molecules (for example, redox label molecules) to be detected can be applied to the various segments of the first electrode 301. When a molecule to be detected pass through the various sections of the first electrode 301, time information that the molecule to be detected moves from one segment of the first electrode 301 to another, in addition to current signal information of the molecule to be detected, can be also obtained, which is helpful to improve the detection resolution of different molecules to be detected.

In another implementation, the first electrode 301 comprises a plurality of segments separated from each other, and the second electrode 303 also comprises a plurality of segments separated from each other, each segment of the first electrode 301 is arranged opposite to one segment of the second electrode 303. Each segment of the first electrode 301 can be supported by a corresponding first supporting element 201, and each segment of the second electrode 303 can be supported by a corresponding second supporting element 304. In this case, different voltages can be applied to each segment of the first electrode 301 and the segment of the second electrode 303 arranged opposite to this segment of the first electrode 301, when a molecule to be detected passes through various electrode segments sequentially under the control of a guiding electric field, only the electrode segments applied with a voltage therebetween corresponding to a redox potential window of the molecule can detect a current signal. Thus, different molecules to be detected can be detected by different segments of the first electrode 301 and the corresponding segments of the second electrode 303.

In still another implementation, the first electrode 301 may comprise one segment, and the second electrode 303 may comprise a plurality of segments separated from each other. In this case, a voltage can be applied to the first electrode 301, and voltages corresponding to the redox windows of different molecules to be detected (for example, redox label molecules) can be applied to the various segments of the second electrode 303. When a molecule to be detected pass through the various segments of the second electrode 303, time information that the molecule to be detected move from one segment of the second electrode 303 to another, in addition to current signal information of the molecule to be detected, can be also obtained, which is helpful to improve the detection resolution of different molecules to be detected.

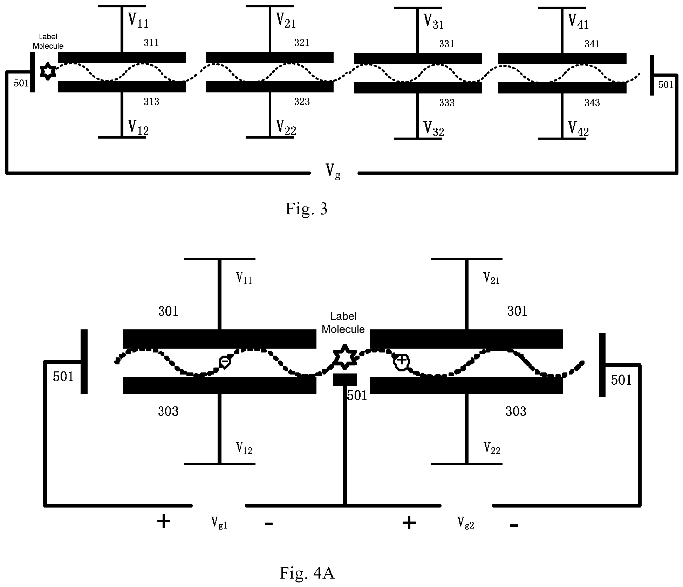

Fig. 3 is a schematic diagram showing a microwell electrode according to an embodiment of the present invention, wherein the first electrode and the second electrode each includes four segments. As shown in Fig. 3, the first electrode 301 comprises a first segment 311 of the first electrode, a second segment 321 of the first electrode, a third segment 331 of the first electrode and a fourth segment 341 of the first electrode. Voltages applied to the first segment 311 of the first electrode to the fourth segment 341 of the first electrode are V₁₁, V₂₁, V₃₁, and V₄₁, respectively. The second electrode 303 comprises a first segment 313 of the second electrode, a second segment 323 of the second electrode, a third segment 333 of the second electrode and a fourth segment 343 of the second electrode. Voltages applied to the first segment 313 of the second electrode to the fourth segment 343 of the second electrode are V₁₂, V₂₂, V₃₂, and V₄₂, respectively. Each segment of the first electrode 301 is arranged opposite to one segment of the second electrode 303, and each segment of the first electrode 301 and the segment of the second electrode 303 arranged opposite thereto are used as a set of detection electrodes. For example, the first segment 311 of the first electrode is arranged opposite to the first segment 313 of the second electrode, and these two segments are used as a set of detection electrodes. A detection voltage applied on each set of detection electrodes corresponds to a label molecule to be detected. A voltage difference between the two guiding electrodes 501 is V_{g}. When a label molecule to be detected passes through the four sets of detection electrodes sequentially under the control of the guiding electric field of guiding electrodes 501, only a set of detection electrodes that is applied with a detection voltage corresponding to the redox window of the label molecule to be detected can detect a current signal. Thus, after setting detection voltages in advance, the type of a label molecule to be detected can be determined according to the detection electrode that detects a current signal. For example, a detection voltage applied on the first set of detection electrodes corresponds to a redox window of a certain label molecule to be detected, upon detecting a current signal by the first set of detection electrodes, it can be determined that the label molecule to be detected has been detected.

The present invention further provides a microwell electrode array, comprising one or more microwell electrodes according to any one of the above embodiments. In an embodiment, a plurality of microwell electrodes may share a guiding electrode.

Fig. 4A is a schematic diagram showing a microwell electrode array consisting of two microwell electrodes. As shown in Fig. 4A, the microwell electrode comprises a first microwell electrode and a second microwell electrode, the first microwell electrode and the second microwell electrode sharing an intermediate guiding electrode 501. The voltages applied on the first electrode 301 and the second electrode 303 of the first microwell electrode are V₁₁ and V₁₂, respectively; and the voltages applied on the first electrode 301 and the second electrode 303 of the second microwell electrode are V₂₁ and V₂₂, respectively. A voltage difference V_{g1} is applied on the two guiding electrodes 501 of the first microwell electrode, and a voltage difference V_{g2} is applied on the two guiding electrodes 501 of the second microwell electrode. A negatively charged label molecule is drawn into the channel of the first microwell electrode, and a positively charged label molecule is drawn into the channel of the second microwell electrode, thus achieving screening of label molecules. In this way, a microwell electrode array consisting of two microwell electrodes can recognize the charge polarity of a label molecule and can determine the type of the label molecule according to its current signal.

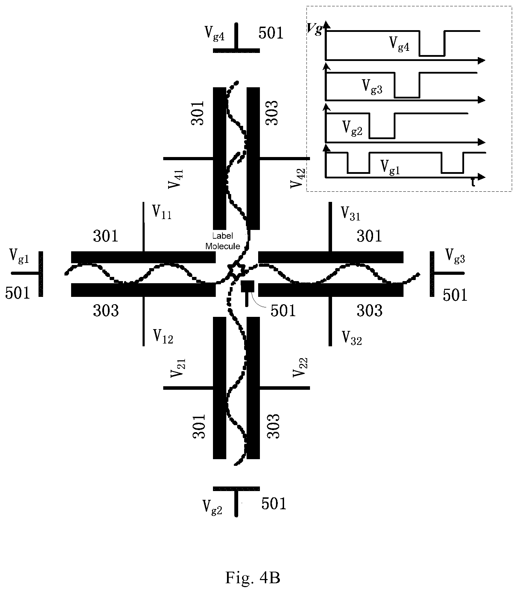

Fig. 4B is a schematic diagram showing a microwell electrode array consisting of four microwell electrodes. As shown in Fig. 4B, the microwell electrode comprises a first microwell electrode, a second microwell electrode, a third microwell electrode and a fourth microwell electrode. These four microwell electrodes share an intermediate guiding electrode 501. The voltages applied on the first electrode 301 and the second electrode 303 of the first microwell electrode are V₁₁ and V₁₂, respectively; the voltages applied on the first electrode 301 and the second electrode 303 of the second microwell electrode are V₂₁ and V₂₂, respectively; the voltages applied on the first electrode 301 and the second electrode 303 of the third microwell electrode are V₃₁ and V₃₂, respectively; and the voltages applied on the first electrode 301 and the second electrode 303 of the fourth microwell electrode are V₄₁ and V₄₂, respectively. Voltage differences applied on the guiding electrodes 501 of the first to fourth microwell electrodes are V_{g1}, V_{g2}, V_{g3} and V_{g4}, respectively. A schematic view in the dashed box shows variations of V_{g1}, V_{g2}, V_{g3} and V_{g4} over time. With a periodic guiding electric field, different label molecules can be drawn into the channels of the four microwell electrodes at different timings during a timing period, which may effectively increase the reaction and detection time of each label molecule in the channel, so that the detection signal of the label molecule can be effectively enhanced, the signal-to-noise ratio of the detection can be improved, and the signal interference between adjacent label molecules can be avoided.

A plurality of microwell electrodes in a microwell electrode array may be arranged in different shapes.

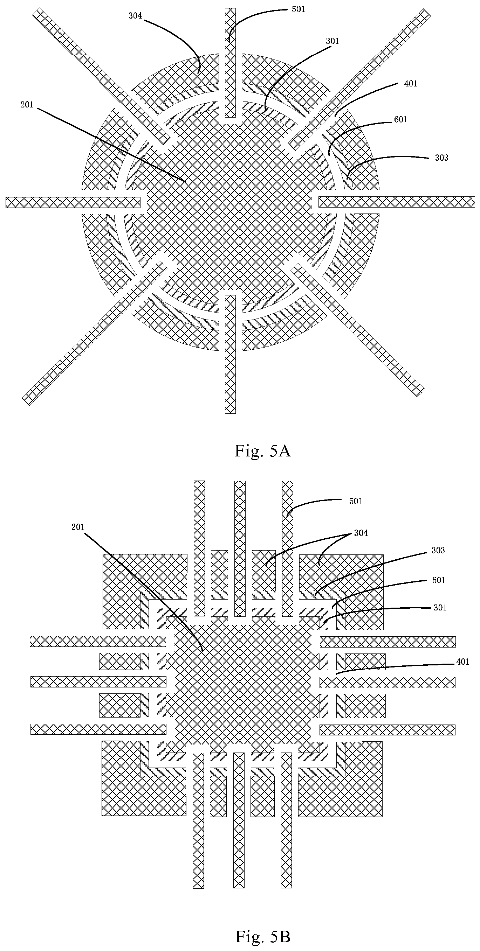

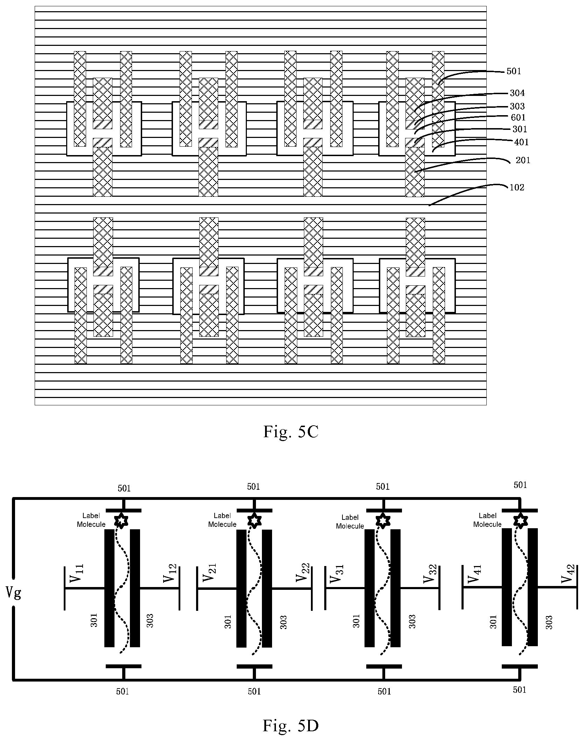

Figs. 5A, 5B, 5C and 5D show four arrangements of a plurality of microwell electrodes in a microwell electrode array, respectively. In Fig. 5A, the plurality of microwell electrodes are arranged in a ring shape. In Fig. 5B, the plurality of microwell electrodes are arranged in a square or rectangular shape. In Fig. 5C, the plurality of microwell electrodes are arranged as a matrix of rows and columns. However, the present invention is not limited to the above arrangements. For example, the plurality of microwell electrodes may also be arranged in an elliptical, a circular, a fan, a zigzag, or a gear shape, etc. Further, the plurality of microwell electrodes is not limited to have a two dimensional arrangement, for example, the plurality of microwell electrodes may also be arranged as laminated layers which is a three dimensional arrangement.

Further, the plurality of microwell electrodes in the microwell electrode array may also be connected in series, i.e., the channels of the various microwell electrodes connect with each other in series, as shown in Figs. 5A or 5B. The plurality of microwell electrodes may also be independent from each other as shown in Fig. 5C, in which case, the microwell electrodes each may be used to detect different label molecules. Alternatively, the plurality of microwell electrodes may also be connected in parallel, i.e., the channels of the various microwell electrodes are connected with each other in parallel, as shown in Fig. 5D, all the guiding electrodes of the plurality of (for example, 4) microwell electrodes have a voltage difference V_{g} applied thereon, the plurality of microwell electrodes can detect label molecules in parallel with their respective detection voltages (i.e., the voltages applied to the first electrode and the second electrode).

The present invention further provides a sensor chip comprising the microwell electrode array according to the above embodiment. The sensor chip, in an embodiment, may comprise a microwell electrode array, and may further comprise a circuit for applying voltages to the electrodes (for example, a first electrode, a second electrode, or a guiding electrode), an amplification device, a current sensing circuit, a voltage sensing circuit, etc.

The present invention further provides a sequencing system comprising the sensor chip of the above embodiment.

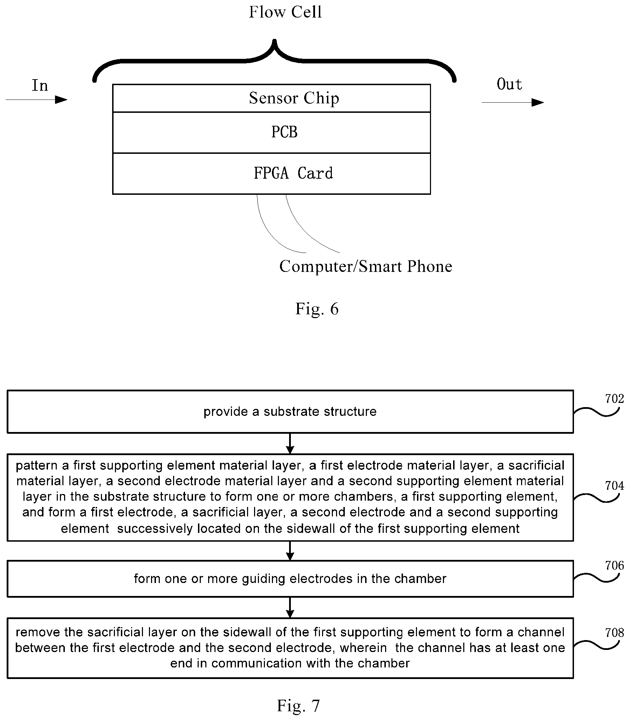

Fig. 6 is a schematic diagram showing a sequencing system according to an embodiment of the present invention. As shown in Fig. 6, the sequencing system comprises a flow cell, a sensor chip, a printed circuit board (PCB), a Field Programmable Gate Array (FPGA) card and a computer/mobile device. The flow cell may supply a microfluid to the sensor chip. A packaged sensor chip may be mounted on the PCB board by means of a socket on the PCB board. The PCB board may comprise a current and/or voltage sensing circuit chip, a current and/or voltage amplification circuit chip, a data storage chip, a system control chip, etc. The FPGA card can be integrated with the PCB board, and can be connected to the computer/mobile device via an interface, so that it is possible to develop application control software on the computer or mobile device to control the sequencing system.

In a practical application, a sequencing system can be designed in different forms, for example, a palmtop form, a laptop form, a benchtop form, or a mainframe form. A microwell electrode array may be integrated into a sensor chip of a sequencing system and software programming can be performed by a FPGA card, thus achieving the integration of hardware and software.

The microwell electrode proposed in the present invention can be compatible with CMOS process. Below, an exemplary method for manufacturing a microwell electrode will be introduced.

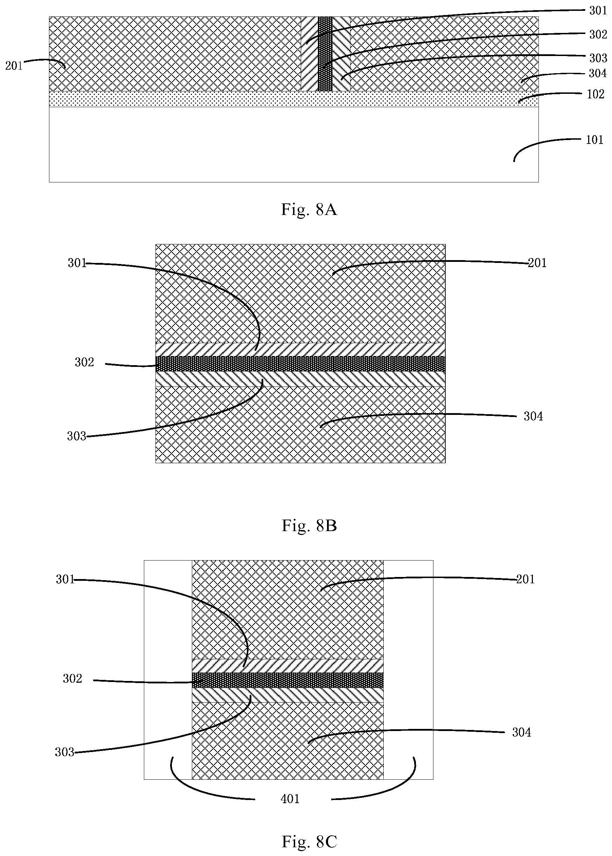

Fig. 7 is a schematic diagram showing a simplified flow of a method for manufacturing a microwell electrode according to an embodiment of the present invention. Figs. 8A-8K show pruducts at various stages of a method for manufacturing a microwell electrode according to an embodiment of the present invention. The method for manufacturing the microwell electrode will be described below in detail with reference to Figs. 7 and 8A-8K.

First, in step 702, a substrate structure is provided.

Fig. 8A shows a sectional view of a substrate structure according to an embodiment of the present invention. Fig. 8B shows a top view of the substrate structure shown in Fig. 8A. As shown in Figs. 8A and 8B, the substrate structure comprises a substrate 101 with an insulating layer 102 on its surface and a first supporting element material layer 201 on the insulating layer 102. Moreover, the first supporting element material layer 201 has successively on its sidewall (for example, the sidewall(s) at one side or both sides of the first supporting element material layer 201) a first electrode material layer 301, a sacrificial material layer 302, a second electrode material layer 303 and a second supporting element material layer 304. Note that, in the substrate structure shown in Figs. 8A and 8B, the first supporting element material layer 201 has on its sidewall at one side a first electrode material layer 301, a sacrificial material layer 302, a second electrode material layer 303 and a second supporting element material layer 304. In some embodiments, the first supporting element material layer 201 may also have on its sidewall at the other side a first electrode material layer 301, a sacrificial material layer 302, a second electrode material layer 303 and a second supporting element material layer 304.

As a non-limiting example, the first electrode material layer 301 may have a thickness of about 1-1000nm, for example, 30nm, 50nm, 200nm, 600nm, 800nm or the like. The sacrificial material layer 302 may have a thickness of about 0.5-100nm, for example, 5nm, 10nm, 20nm, 40nm, 80nm or the like. The second electrode material layer 303 may have a thickness of about 1-1000nm, for example, 30nm, 50nm, 200nm, 600nm, 800nm or the like.

The first electrode material layer 301 and the second electrode material layer 303 may be constituted by the same material or different materials. Further, the sacrificial material layer 302 can be formed by a material which can be selected according to the first electrode material layer 301 and the second electrode material layer 303. Exemplarily, the first electrode material layer 301 and the second electrode material layer 303 may be formed by a material selected from a group consisting of silicon, platinum, gold, silver, indium tin oxide, carbon-based materials (for example, diamond, graphite, amorphous carbon, etc), or any combination thereof. The sacrificial material layer 302 may be formed by a material selected from a group consisting of chromium, tungsten, aluminum, aluminum oxide, silicon, silicon oxide, silicon nitride or any combination thereof. In a specific embodiment, the first electrode material layer 301 and the second electrode material layer 303 may be formed by platinum, and the sacrificial material layer 302 may be formed by chromium or a silicon oxide. In another specific embodiment, the first electrode material layer 301 and the second electrode material layer 303 may be formed by gold, and the sacrificial material layer 302 may be formed by tungsten.

Next, in step 704, the first supporting element material layer 201, the first electrode material layer 301, the sacrificial material layer 302, the second electrode material layer 303 and the second supporting element material layer 304 are patterned to form one or more chambers 401, a first supporting element 201, and form a first electrode 301, a sacrificial layer 302, a second electrode 303 and a second supporting element 304 successively located on the sidewall of the first supporting element 201, as shown in Fig. 8C. Herein, the first supporting element 201, the first electrode 301, the sacrificial layer 302, the second electrode 303 and the second supporting element 304 are formed from the first supporting element material layer, the first electrode material layer, the sacrificial material layer, the second electrode material layer and the second supporting element material layer, respectively. The formed chamber 401 communicates with a channel 601 that is formed subsequently. Further, in this step, it is also possible to separate the first electrode material layer 301 and/or the second electrode material layer 303 into a plurality of segments, so that the formed first electrode 301 and/or the second electrode 303 each comprise a plurality of segments separated from each other.

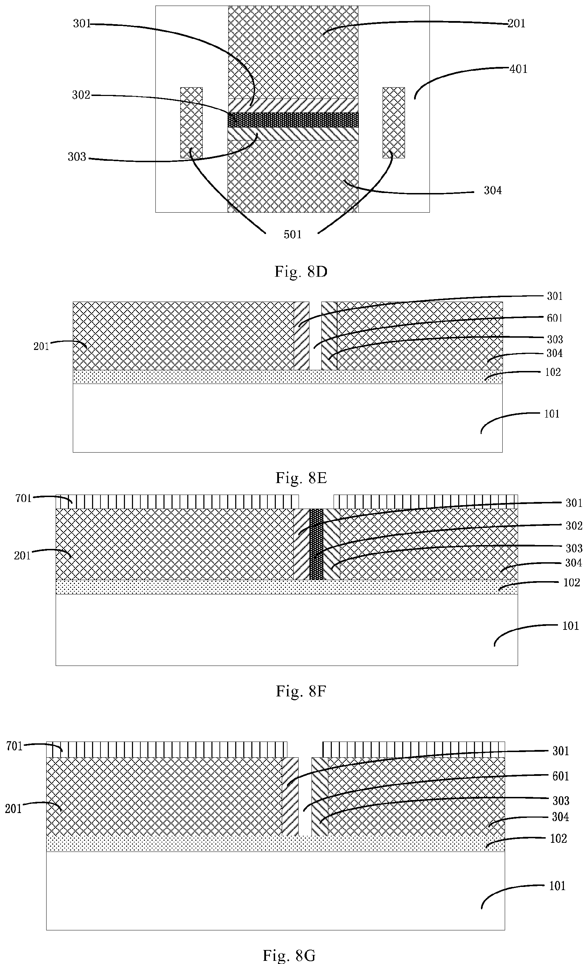

Then, in step 706, one or more guiding electrode(s) 501 are formed in the chamber 401, as shown in Fig. 8D. For example, the guiding electrode 501 may be formed by processes such as photolithography, deposition, and peeling. The guiding electrode 501 may also be formed by processes such as deposition, planarization, and etching. Preferably, the guiding electrode 501 may be substantially perpendicular to the first electrode 301. Alternatively, the guiding electrode 501 may be substantially perpendicular to the second electrode 303. That is, the electric field direction between the guiding electrodes 501 is substantially perpendicular to the electric field direction between the first electrode 301 and the second electrode 303 after applying voltages to the guiding electrodes 501, the first electrode 301 and the second electrode 302.

Then, in step 708, the sacrificial layer 302 on the sidewall of the first supporting element 201 is removed to form a channel 601 between the first electrode 301 and the second electrode 303, wherein the channel 601 has at least one end in communication with the chamber 401, as shown in Fig. 8E.

In an embodiment, before removing the sacrificial layer 302 from the sidewall of the first supporting element 201, a passivation layer 701 may be formed on the surface of at least one of the first supporting element 201, the second supporting element 304, the first electrode 301, or the second electrode 303, as shown in Fig. 8F. Exemplarily, the passivation layer 701 may be formed by a silicon nitride, a silicon oxide and the like. For example, a passivation material can be deposited on the top of the first supporting element 201, the first electrode 301, the second electrode 303 and the second supporting element 304. Then, the deposited passivation material can be patterned to form a passivation layer 701 on the surface of at least one of the first supporting element 201, the second supporting element 304, the first electrode 301, or the second electrode 303. Then, the sacrificial layer 302 may be removed by a selective etching process to form a channel 601 between the first electrode 301 and the second electrode 303, as shown in Fig. 8G.

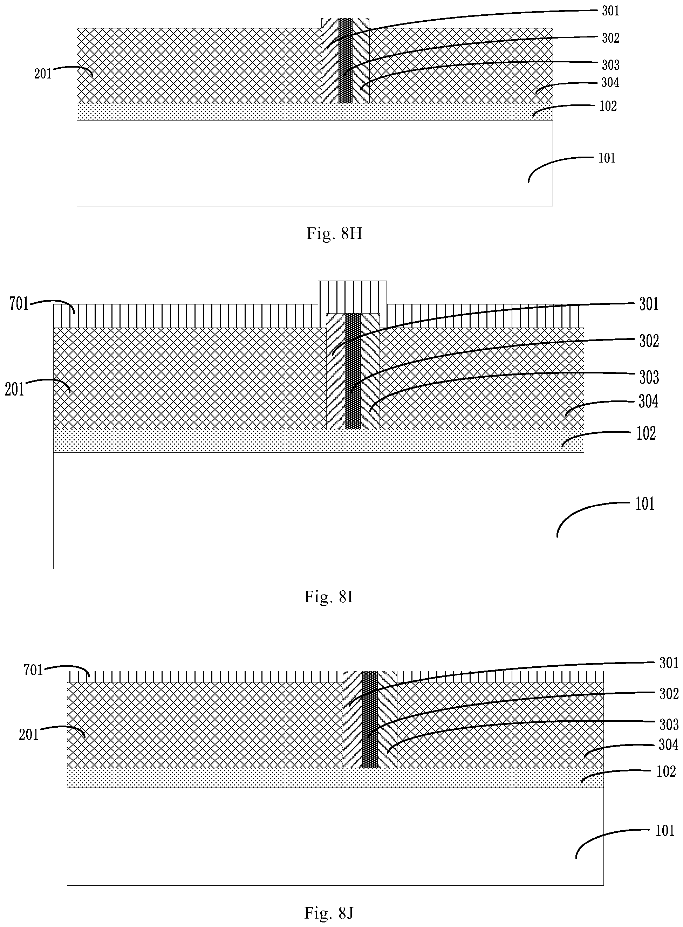

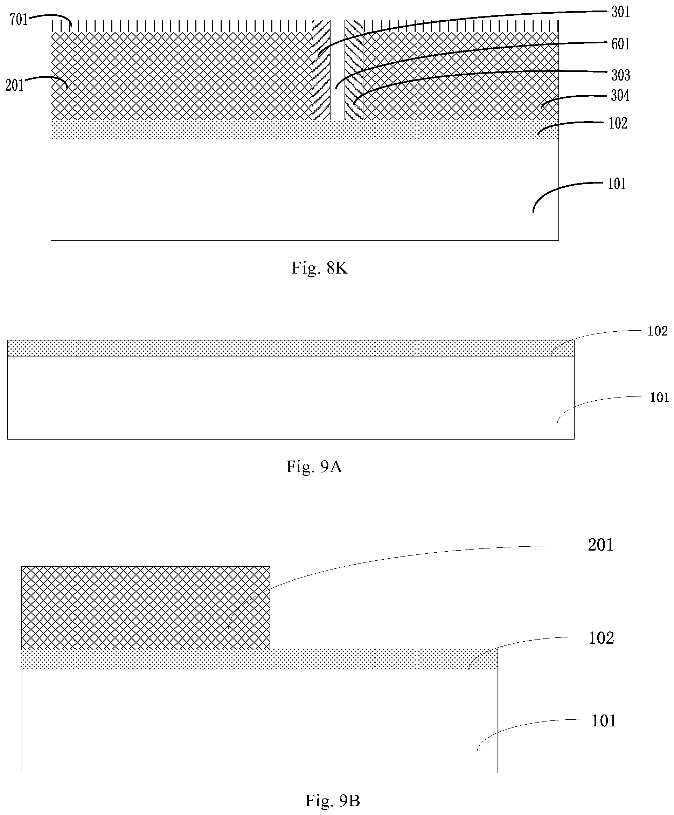

In another embodiment, before removing the sacrificial layer 302 from the sidewall of the first supporting element 201, a portion of the top of the first supporting element 201 and a portion of the top of the second supporting element 304 can be removed to expose a portion of the first electrode 301, a portion of the sacrificial layer 302 and a portion of the second electrode 303, as shown in Fig. 8H. Then, a passivation layer 701 is deposited on the remaining portion of the first supporting element 201, the remaining portion of the second supporting element 304, the exposed portion of the first electrode 301, the exposed portion of the sacrificial layer 302 and the exposed portion of the second electrode 303, as shown in Fig. 8I. Thereafter, the deposited passivation layer 701 is planarized to form a passivation layer 701 on the remaining portion of the first supporting element 201 and the remaining portion of the second supporting element 304, and expose the sacrificial layer 302, as shown in Fig. 8J. In one implementation, the planarization process may stop at the upper surface of the first electrode 301, the upper surface of the sacrificial layer 302, and the upper surface of the second electrode 303. Alternatively, the planarization process may remove a portion of the first electrode 301, a portion of the sacrificial layer 302 and a portion of the second electrode 303, so long as a portion of the passivation layer 701 is remained on the remaining portion of the first supporting element 201 and the remaining portion of the second supporting element 304. Then, the exposed sacrificial layer 302 may be removed by a selective etching process to form a channel 601 between the first electrode 301 and the second electrode 303, as shown in Fig. 8K.

As an example, refering to Figs. 1A and 1B, the formed channel 601 may have a width W of about 0.5-100nm (for example, 1nm, 2nm, 10nm, 50nm, 80nm, etc.), the channel 601 may have a length L of about 50nm-100µm (for example,100nm, 500nm, 5µm, 10µm, 30µm, etc.), and the channel 601 may have a depth H of about 0-10µm (for example, 100nm, 300nm,1µm, 2µm, 8µm, etc.) Herein, the width of the channel 601 is the distance between the first electrode 301 and the second electrode 303, the length of the channel 601 is the length extending around the first supporting element 201, and the depth of the channel 601 is the distance between the upper surface of the first electrode 301/second electrode 303 and the insulating layer 102.

With the above method, a microwell electrode shown in Figs. 1A, 1B and 1C is formed. However, it should be understood that the present invention is not limited to the above method for manufacturing a microwell electrode. In practical applications, a method for manufacturing a microwell electrode can be adopted based on the specific structure of the microwell electrode, for example, some steps may be added on the basis of the above method, or some steps of the above method can be adjusted. For example, when forming a first electrode material layer over the sidewall of a first supporting element, it can be determined that whether the first electrode material layer will be formed over a sidewall(s) at one side or both sides of the first supporting element based on the required number of the first electrodes.

After forming a microwell electrode, a nanostructure 801 capable of immobilizing an enzyme or a chemical substance 802 to be detected may be formed on the bottom or a sidewall of the chamber 401, or on the bottom or a sidewall of the channel 601, or on the guiding electrode 501. Fig. 1C shows a situation in which a nanostructure is located on the bottom of the chamber 401. In an embodiment, the nanostructure 801 may be a nanodot. Exemplarily, the size of the nanostructure 801 may be 1-100nm, for example, 8nm, 20nm, 50nm, 80nm and the like. In an embodiment, the nanostructure 801 may be formed by a material selected from a group consisting of a transition metal oxide such as zirconium dioxide (ZrO₂) or hafnium dioxide (HfO₂), an inert metal, an inorganic polymer, an organic polymer, or any combination thereof.

The substrate structure shown in Figs. 8A and 8B can be formed in different ways. Below, a specific implementation of forming the substrate structure will be introduced with reference to Figs. 9A-9J.

First, as shown in Fig. 9A, a substrate 101 with an insulating layer 102 on its surface is provided. The substrate 101 may be, for example, a silicon substrate, a substrate made of a Group III-V semiconductor material, a silicon on insulator (SOI) substrate, or may be an oxide semiconductor substrate such as ZnO, CdO, TiO₂, Al₂O₃, SnO, or may be a substrate made of an insulating material, such as a quartz glass, or a soda glass. The insulating layer 102 may be formed on the substrate 101 by thermal oxidation, or by deposition (e.g., physical vapor deposition (PVD), chemical vapor deposition (CVD), etc.). Typically, the insulating layer 102 may be a silicon dioxide layer.

Next, as shown in Fig. 9B, a first supporting element material layer 201 is formed on a portion of the insulating layer 102. The first supporting element material layer 201 may be formed by a conductive material, or a non-conductive material, preferably, a conductive material. For example, a conductive material may be deposited on the insulating layer 102 through deposition (such as PVD or CVD), and then the conductive material is patterned to form the first supporting element material layer 201. As a non-limiting example, a sectional of the first supporting element material layer 201 in the direction parallel to the surface of the substrate 101 is elliptical, square, circular, or polygonal. However, it should be understood that the present invention is not limited thereto, and the first supporting element material layer 201 may also have other suitable shapes.

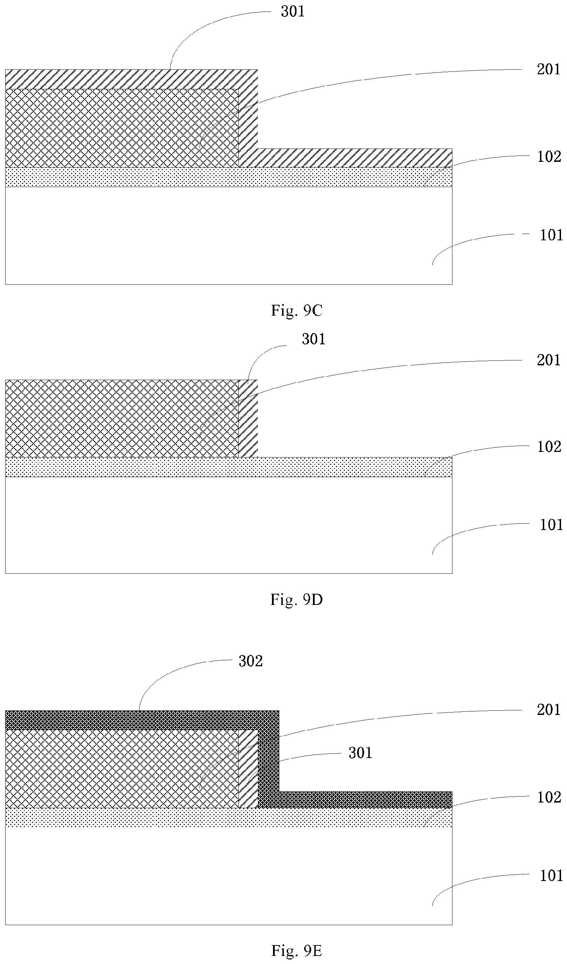

Then, as shown in Fig. 9C, a first electrode material layer 301 is deposited to cover the upper surface and a sidewall of the first supporting element material layer 201. Herein, the first electrode material layer 301 may also cover all or a portion of the exposed potion of the insulating layer 102.

Then, as shown in Fig. 9D, the first electrode material layer 301 on the upper surface of the first supporting element material layer can be removed by anisotropic dry etching, for example reactive ion etching (RIE), ion beam etching (IBE), only preserving the first electrode material layer 301 on the sidewall of the first supporting element material layer 201. In an embodiment, in the case of also depositing the first electrode material layer 301 on the insulating layer 102, the first electrode material layer 301 on the insulating layer 102 is removed.

Next, as shown in Fig. 9E, a sacrificial material layer 302 is deposited to cover the upper surface of the first supporting element material layer 201, and the upper surface and the sidewall of the remaining first supporting element material layer 301 (that is, the first electrode material layer 301 on the sidewall of the first supporting element material layer 201). Herein, the sacrificial material layer 302 may also cover all or a portion of the exposed potion of the insulating layer 102.

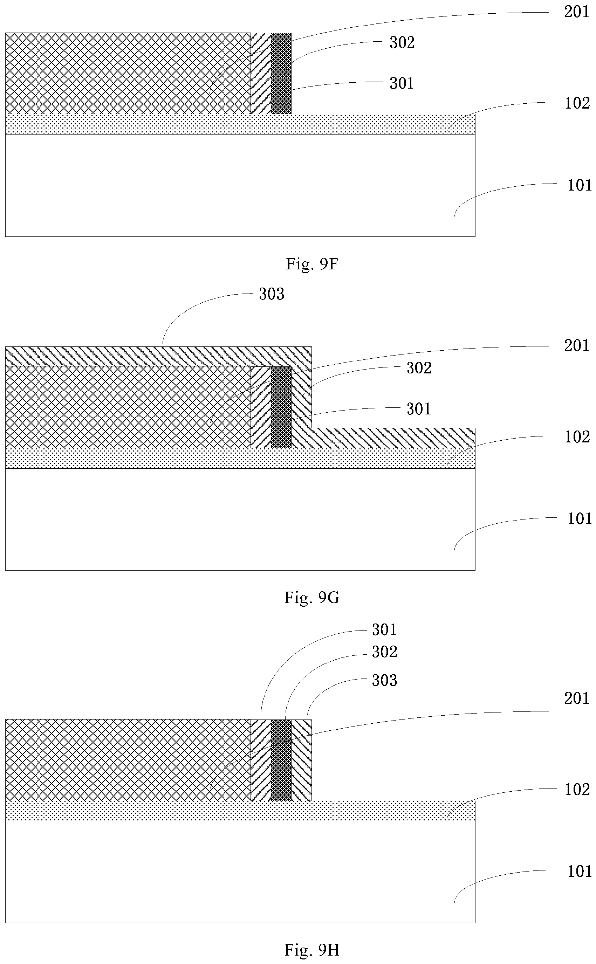

Next, as shown in Fig. 9F, the sacrificial material layer 302 on the upper surface of the first supporting element material layer 201 and the upper surface of the remaining first electrode material layer 301 is removed, only preserving the sacrificial material layer 302 on the sidewall of the remaining first electrode material layer 301. In one embodiment, in the case of also depositing the sacrificial material layer 302 on the insulating layer 102, the sacrificial material layer 302 on the insulating layer 102 is removed.

Then, as shown in Fig. 9G, a second electrode material layer 303 is deposited to cover the upper surface of the first supporting element material layer 201, the upper surface of the remaining first electrode material layer 301 and the upper surface and the sidewall of the remaining sacrificial material layer 302. Herein, the second electrode material layer 303 may also cover all or a portion of the exposed potion of the insulating layer 102.

Then, as shown in Fig. 9H, the second electrode material layer 303 on the upper surface of the first supporting element material layer 201, the upper surface of the remaining first electrode material layer 301, the upper surface of the remaining sacrificial material layer 302 is removed, persevering the second electrode material layer 303 on the sidewall of the remaining sacrificial material layer 302. In one embodiment, in the case of also depositing the second electrode material layer 303 on the insulating layer 102, the second electrode material layer 303 on the insulating layer 102 is removed.

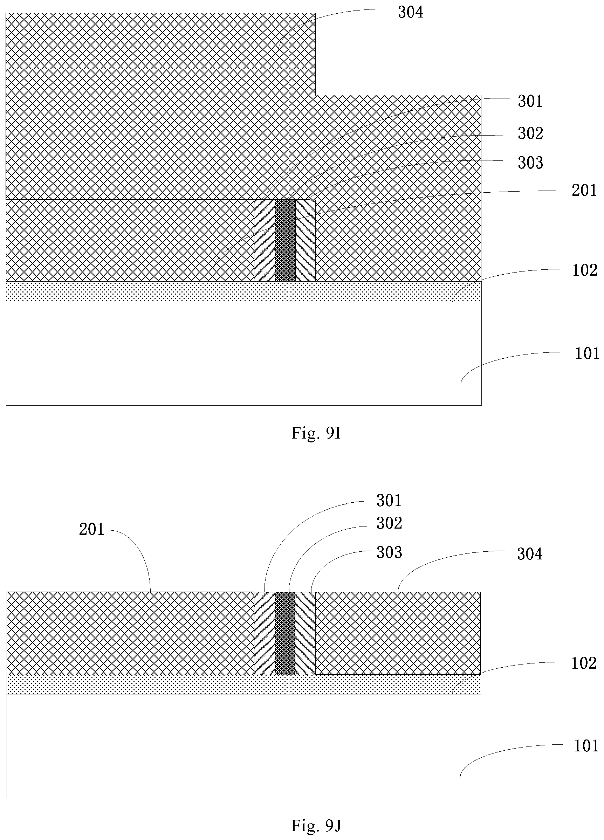

Then, as shown in Fig. 9I, a second supporting element material layer 304 is deposited to cover the first supporting element material layer 201, the first electrode material layer 301 on the sidewall of the first supporting element material layer 201, the sacrificial material layer 302 over the sidewall of the first supporting element material layer 201, the second electrode material layer 303 over the sidewall of the first supporting element material layer 201 and the portion which is not covered of insulating layer 102.

Then, as shown in Fig. 9J, the deposited second supporting element material layer 304 is planarized to expose the sacrificial material layer 302 over the sidewall of the first supporting element material layer 201 to form a substrate structure. In one implementation, this planarization process may make the upper surfaces of the first supporting element material layer 201, the first electrode material layer 301, the sacrificial layer 302, and the second electrode material layer 303 over the sidewall of the first supporting element material layer 201, and the upper surface of the second supporting element material layer 304 substantially flush.

Subsequent steps 704-708 can be performed according to the method described above after forming the substrate structure according to Figs. 9A to 9J, which will not be repeated herein.

In an embodiment, the label molecule for use in a circular redox reaction may be, for example, selected from:

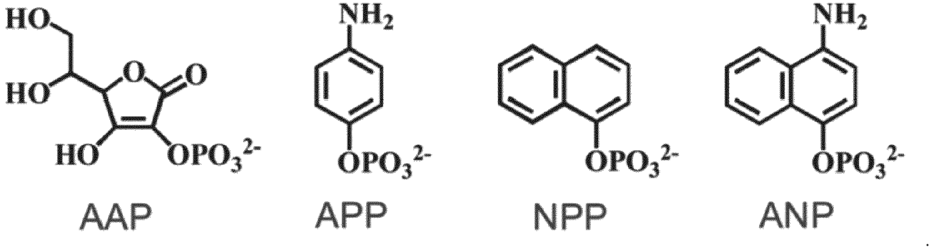

In an embodiment, the label molecule-modified dNTP or an analogue thereof may be, for example, synthesized by the following method:

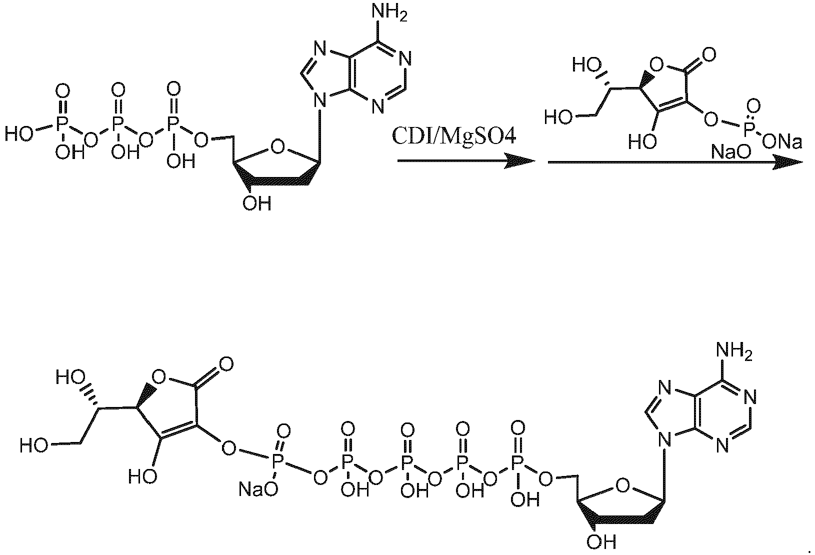

In the invention, different label molecules may be designed to modify four different dNTP molecules, NTP molecules or analogues thereof, whereby different free label molecules have different redox potentials, and further the different dNTP molecules, NTP molecules or analogues can be distinguished. In an embodiment, the different label molecules may be shown as follows:

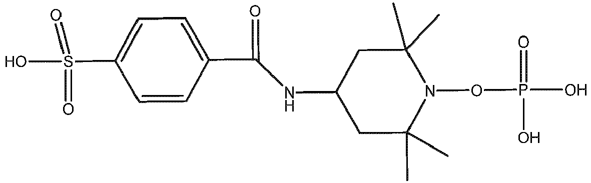

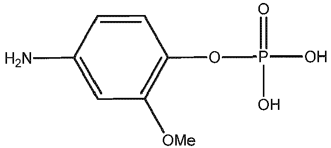

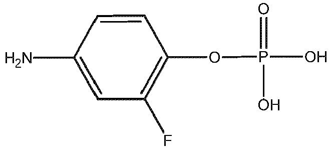

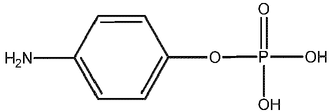

When the number of charges carried by different free label molecules is different, they have different migration speeds under the action of a guiding electrode; in an embodiment, the charges carried by different label molecules are as follows:

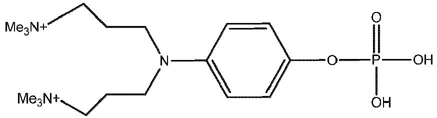

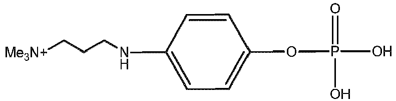

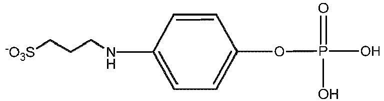

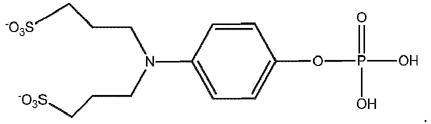

In an embodiment, non-charged or negatively charged dNTP modified with a label molecule releases a positively charged redox active substance in the presence of DNA polymerase and alkaline phosphatase, as shown below:

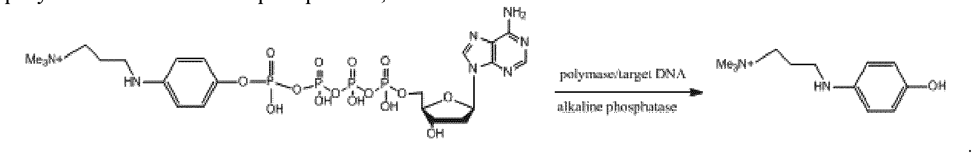

In an embodiment, the principle for the circular redox reaction occurred between the first electrode and the second electrode is as follows:

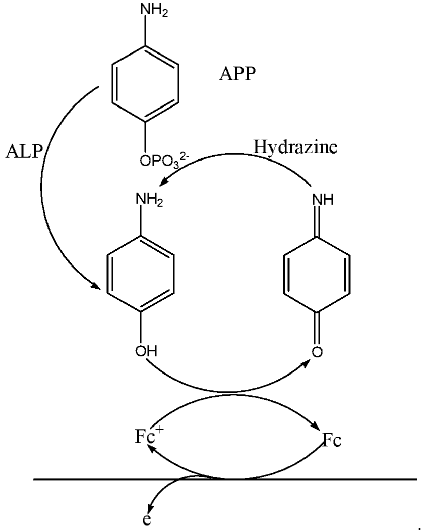

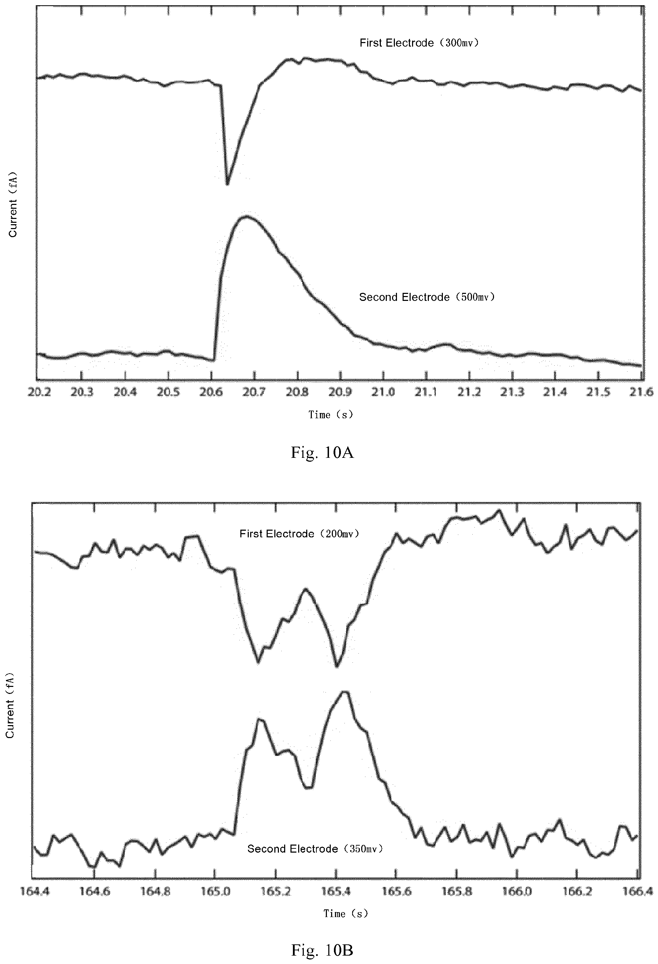

Fig. 10A and Fig. 10B show the detection results of different free label molecules detected by using an exemplary microwell electrode of the invention, respectively, wherein Fig. 10A is a redox current curve of hexacyanoferrate molecule detected by the microwell electrode of the invention, and Fig. 10B is a redox current curve of ferrocene molecule detected by the microwell electrode of the invention. The results in Fig. 10A and Fig. 10B show that hexacyanoferrate molecule and ferrocene molecule are quite different from each other in terms of redox potential window and curve shape. The results show that various free label molecules may be distinguished and identified by the current signals detected by the microwell electrode of the invention. When each of the basic units (such as dNTP, NTP or an analogue thereof) in a reaction solution is modified with a different label molecule, the microwell electrode of the invention may be used to distinguish and identify the type of the label molecule by the detected unique electric signal, further distinguish and identify the type of the basic unit (such as dNTP, NTP or an analogue thereof) in the reaction solution that is involved in the reaction, and finally achieve analysis of the chemical substance to be tested (such as nucleic acid).

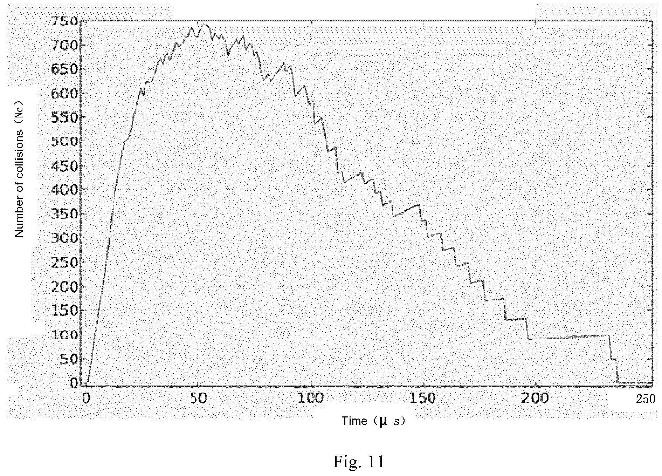

Fig. 11 gives distribution of the number of collisions between a single label molecule and an electrode in an exemplary microwell electrode of the invention, which is obtained by a simulation computation. Without taking into account non-ideal conditions such as molecule absorption, if one electron is exchanged when the electrochemically active molecule collides with the electrode once, the number of collisions within a unit time may be directly converted to the maximum theoretical current value that is generated. Therefore, by means such as further decreasing the minimum size of the channel, changing the chemical structure of the label molecule, increasing the electron exchange number of each collision, and treating the electrode surface so as to reduce molecule absorption, the current signal can be further amplified, so as to enhance the accuracy of the detection of electric signal.

Hereto, a microwell electrode and a method for manufacturing the same, and a method for analysis of a nucleic acid based on the microwell electrode according to embodiments of the present invention have been described in detail. Some details well known in the art are not described to avoid obscuring the concept of the present invention. According to the above description, those skilled in the art would fully know how to implement the technical solutions disclosed herein. Further, the various embodiments taught in this specification can be freely combined. It should be understood by those skilled in the art that various modifications can be made to the embodiments described above without departing from the spirit and scope of the invention as defined by the appended claims.

## Claims

1. A microwell electrode, comprising:
a substrate and an insulating layer on a surface of the substrate;
one or more first electrodes;
one or more second electrodes each arranged opposite to one first electrode, wherein a channel is defined by and located between each first electrode and the second electrode opposite thereto, and the channel has at least one end in communication with a chamber; and
one or more guiding electrodes located in the chamber,
wherein the first electrode, the second electrode and the guiding electrodes are located on a surface of the insulating layer away from the substrate.

2. The microwell electrode according to claim 1, further comprising:
a first supporting element for supporting the one or more first electrodes.

3. The microwell electrode according to claim 1, wherein
the microwell electrode comprises a plurality of first electrodes and a plurality of first supporting elements, each electrode is supported by a corresponding first supporting element; and/or
the microwell electrode comprises a plurality of second electrodes and a plurality of second supporting elements, each second electrode is supported by a corresponding second supporting element.

4. The microwell electrode according to claim 1, wherein
at least one of the first electrode and the second electrode comprises a plurality of segments separated from each other.

5. The microwell electrode according to claim 4, further comprising:
a plurality of first supporting elements, each segment of the first electrode is supported by a corresponding first supporting element; and/or
a plurality of second supporting elements, each segment of the second electrode is supported by a corresponding second supporting element.

6. The microwell electrode according to claim 5, wherein
the first supporting element is a conductive element; and/or
the second supporting element is a conductive element.

7. The microwell electrode according to claim 1, further comprising:
a nanostructure capable of immobilizing an enzyme or a chemical substance to be detected, wherein the nanostructure is located on the bottom or a sidewall of the chamber, or on the bottom or a sidewall of the channel, or on the guiding electrodes.

8. The microwell electrode according to claim 1, wherein
the channel has a width of 0.5-100nm; and/or
the channel has a length of 50nm-100µm; and/or
the channel has a depth of 0-10µm; and/or
the first electrode has a thickness of 1-1000nm; and/or
the second electrode has a thickness of 1-1000nm.

9. The microwell electrode according to claim 1, further comprising:
a passivation layer located on the surface of the first electrode and/or the second electrode.

10. A microwell electrode array, comprising: the microwell electrode according to any one of claims 1 to 9.

11. The microwell electrode array according to claim 10, wherein the microwell electrode array comprises a plurality of microwell electrodes,
the plurality of microwell electrodes are arranged in an elliptical, a circular, an annular, a fan, a rectangular, a square, a zigzag, or a gear shape, or as a matrix of rows and columns, or as laminated layers.

12. The microwell electrode array according to claim 11, wherein
more than one microwell electrode share one guiding electrode.

13. A sensor chip, comprising: the microwell electrode array according to any one of claims 10 to 12.

14. A sequencing system, comprising: the sensor chip according to claim 13.

15. A method for manufacturing a microwell electrode, comprising:
providing a substrate structure comprising a substrate with an insulating layer on its surface and a first supporting element material layer on the insulating layer, wherein the first supporting element material layer has successively on its sidewall a first electrode material layer, a sacrificial material layer, a second electrode material layer and a second supporting element material layer;
patterning the first supporting element material layer, the first electrode material layer, the sacrificial material layer, the second electrode material layer and the second supporting element material layer to form one or more chambers, a first supporting element, and form a first electrode, a sacrificial layer, a second electrode and a second supporting element successively located on the sidewall of the first supporting element;
forming one or more guiding electrodes in the chamber;
removing the sacrificial layer on the sidewall of the first supporting element to form a channel between the first electrode and the second electrode,
wherein the channel has at least one end in communication with the chamber.

16. The method according to claim 15, wherein the step of providing the substrate structure comprises:
providing a substrate with an insulating layer on its surface;
forming a first supporting element material layer on a portion of the insulating layer;
depositing a first electrode material layer to cover the upper surface and a sidewall of the first supporting element material layer;
removing the first electrode material layer on the upper surface of the first supporting element material layer;
depositing a sacrificial material layer to cover the upper surface of the first supporting element material layer, the upper surface and a sidewall of the remaining first electrode material layer;
removing the sacrificial material layer on the upper surface of the first supporting element material layer and the upper surface of the remaining first electrode material layer;
depositing a second electrode material layer to cover the upper surface of the first supporting element material layer, the upper surface of the remaining first electrode material layer and a upper surface and a sidewall of the remaining sacrificial material layer;
removing the second electrode material layer on the upper surface of the first supporting element material layer, the upper surface of the remaining first electrode material layer, and the upper surface of the remaining sacrificial material layer;
depositing a second supporting element material layer to cover the first supporting element material layer, the first electrode material layer on the sidewall of the first supporting element material layer, the sacrificial material layer over the sidewall of the first supporting element material layer, the second electrode material layer over the sidewall of the first supporting element material layer, and the portion which is not covered of the insulating layer;
planarizing the deposited second supporting element material layer to expose the sacrificial material layer over the sidewall of the first supporting element material layer.

17. The method according to claim 15, wherein before removing the sacrificial layer, the method further comprises:
forming a passivation layer on a surface of at least one of the first supporting element, the second supporting element, the first electrode, or the second electrode.

18. The method according to claim 15, wherein before removing the sacrificial layer, the method further comprises:
removing a portion of the top of the first supporting element and a portion of the top of the second supporting element to expose a portion of the first electrode, a portion of the sacrificial layer and a portion of the second electrode;
depositing a passivation layer on the remaining first supporting element, the remaining second supporting element, the exposed portion of the first electrode, the exposed portion of the sacrificial layer and the exposed portion of the second electrode;
planarizing the deposited passivation layer to form a passivation layer on the remaining portion of the first supporting element and the remaining portion of the second supporting element, and expose the sacrificial layer.

19. The method according to claim 15, wherein the step of patterning comprises:
separating the first electrode material layer and/or the second electrode material layer into a plurality of segments, so that the first electrode and/or the second electrode formed each comprises a plurality of segments separated from each other.

20. The method according to claim 15, wherein the method further comprises:
forming a nanostructure capable of immobilizing an enzyme or a chemical substance to be detected on the bottom or a sidewall of the chamber, or on the bottom or a sidewall of the channel, or on the guiding electrodes.

21. The method according to claim 15, wherein
the channel has a width of 0.5-100nm; and/or
the channel has a length of 50nm-100µm; and/or
the channel has a depth of 0-10µm; and/or
the first electrode has a thickness of 1-1000nm; and/or
the sacrificial layer has a thickness of 0.5-100 nm; and/or
the second electrode has a thickness of 1-1000nm.

22. The method according to claim 15, wherein
the first supporting element comprises a conductive element; and/or
the second supporting element comprises a conductive element.

23. A method for analysis of a chemical substance, comprising the steps of:
(1) providing the microwell electrode according to any one of claims 1-9 or the microwell electrode array according to any one of claims 10-12;
(2) adding a reaction solution containing a chemical substance to be tested to the microwell electrode or microwell electrode array, and subjecting the reaction solution to a reaction to produce a charged molecule;
(3) allowing the charged molecule to enter the channel under the action of the guiding electrode and/or a hydromechanics effect, or to be accumulated in the channel under the action of the guiding electrode; and
(4) identifying the type of the charged molecule by using the first electrode, the second electrode and/or the guiding electrode, and therefore obtaining the information of the chemical substance to be tested.

24. The method according to claim 23, wherein in the step (4), the type of the charged molecule is identified with the first electrode, the second electrode and/or the guiding electrode based on one or more effects selected from a group consisting of oxidation-reduction effect, electric resistance effect, capacitance effect, field effect, and tunneling effect.

25. A method for analysis of a nucleic acid molecule, comprising the steps of:
(1) providing the microwell electrode according to any one of claims 1-9 or the microwell electrode array according to any one of claims 10-12;
(2) immobilizing a polymerase (such as DNA polymerase or RNA polymerase) in the chamber or channel of or on the guiding electrode of the microwell electrode or microwell electrode array;
(3) adding to the microwell electrode or microwell electrode array, a reaction solution containing a nucleic acid molecule to be tested, a primer, and at least one (e.g., one, two, three, or four) deoxyribonucleoside triphosphate (dNTP) molecule or nucleoside triphosphate (NTP) molecule or an analogue thereof, wherein the primer can hybridize or anneal to a partial sequence of the nucleic acid molecule to be tested, and each of the at least one dNTP or NTP molecule or analogue is modified with a label molecule, respectively; and later, under a suitable condition, hybridizing the nucleic acid molecule to be tested with the primer to form a complex;
(4) in the presence of the polymerase as a catalyst, incorporating one of the label molecule-modified dNTP or NTP molecule or analogue into the primer, to form an extension product complementary to the nucleic acid molecule to be tested, and removing the label molecule carried by the dNTP or NTP molecule or analogue incorporated into the primer, to provide a free label molecule, wherein the free label molecule is charged;
(5) allowing the free label molecule to enter the channel under the action of the guiding electrode and/or a hydromechanics effect, or to be accumulated in the channel under the action of the guiding electrode; preferably, the free label molecule is controlled to enter or accumulate into different microwell electrode channels by its electrical polarity or release order;
(6) identifying the type of the free label molecule by using the first electrode and the second electrode; and further identifying the type of the dNTP or NTP molecule or analogue incorporated into the primer according to the correspondence between the label molecule and the dNTP or NTP molecule or analogue; and further determining the base at the corresponding position of the nucleic acid molecule to be tested, according to the principle of complementary base pairing; and
(7) repeating the steps (4), (5) and (6) until the extension of the complex is finished.

26. The method according to claim 25, wherein the free label molecule may be a redox active substance that is reactive in a circular redox reaction, or may be converted to a redox active substance that is reactive in a circular redox reaction; preferably, the redox active substance can be subjected to a circular redox reaction between the first electrode and the second electrode, resulting in a detectable current.

27. The method according to claim 25, wherein the reaction solution further comprises a phosphatase.

28. The method according to claim 25, wherein in the step (4), the free label molecule is dephosphorylated in the presence of a phosphatase.

29. The method according to claim 25, wherein the charge carried by the free label molecule is adjusted by selecting a label molecule, so as to adjust the migration speed of the free label molecule under the action of the guiding electrode.

30. The method according to claim 25, wherein in the step (1), the polymerase is immobilized on an insulated layer on the bottom of the chamber or channel, or immobilized on the guiding electrode; preferably, the polymerase is immobilized at a place close to the end of the channel at the bottom of the chamber.

31. The method according to claim 30, wherein a functionalizable region and/or a molecule-binding region is further provided between the insulated layer and the polymerase;
preferably, the functionalizable region comprises silicon dioxide, hafnium oxide, aluminum oxide, tantalum oxide, and/or zirconium oxide; more preferably, the functionalizable material is functionalized with a linking molecule selected from a group consisting of: silicane (e.g., aminopropyltriethoxysilane), thiol (-SH), disulfide (-S-S-), isothiocyanate, alkene and alkyne;
preferably, the molecule-binding region comprises a probe molecule; preferably, the probe molecule is, for example, selected from a group consisting of a biotin, an avidin, an antibody, an antigen, a receptor, a ligand, a DNA sequence, a RNA sequence, a protein and a ligand thereof.

32. The method according to claim 25, wherein in the step (6), the type of the free label molecule is identified by one or more of oxidation-reduction effect, electric resistance effect, capacitance effect, field effect, and tunneling effect.

## Patentansprüche

1. Mikrowellenelektrode, umfassend:
ein Substrat und eine Isolierschicht auf einer Fläche des Substrats,
eine oder mehrere erste Elektrode(n);
eine oder mehrere zweite Elektrode(n), von denen jede einer ersten Elektrode gegenüber angeordnet ist, wobei ein Kanal durch jede erste Elektrode und die zweite, ihr gegenüberliegende Elektrode definiert und dazwischen angeordnet ist und der Kanal wenigstens ein Ende in Verbindung mit einer Kammer aufweist; und
eine oder mehrere Führungselektrode(n) in der Kammer angeordnet ist/sind;
wobei die erste Elektrode, die zweite Elektrode und die Führungselektroden auf einer Fläche der Isolierschicht von dem Substrat abgewandt angeordnet sind.

2. Mikrowellenelektrode nach Anspruch 1, weiterhin umfassend:
ein erstes Trägerelement zum Tragen der einen oder mehreren ersten Elektrode(n).

3. Mikrowellenelektrode nach Anspruch 1, wobei
die Mikrowellenelektrode mehrere erste Elektroden und mehrere erste Trägerelemente umfasst, wobei jede Elektrode von einem entsprechenden ersten Trägerelement getragen ist und/oder
die Mikrowellenelektrode mehrere zweite Elektroden und mehrere zweite Trägerelemente umfasst, wobei jede zweite Elektrode von einem entsprechenden zweiten Trägerelement getragen ist.

4. Mikrowellenelektrode nach Anspruch 1, wobei
Wenigstens entweder die erste Elektrode oder die zweite Elektrode mehrere voneinander getrennte Segmente umfasst.

5. Mikrowellenelektrode nach Anspruch 4, weiterhin umfassend:
mehrere erste Trägerelemente, wobei jedes Segment der ersten Elektrode von einem entsprechenden ersten Trägerelement getragen ist, und/oder
mehrere zweite Trägerelemente, wobei jedes Segment der zweiten Elektrode von einem entsprechenden zweiten Trägerelement getragen ist.

6. Mikrowellenelektrode nach Anspruch 5, wobei
das erste Trägerelement ein leitendes Element ist; und/oder
das zweite Trägerelement ein leitendes Element ist.

7. Mikrowellenelektrode nach Anspruch 1, weiterhin umfassend:
eine Nanostruktur, die zum Festsetzen eines Enzyms oder einer zu detektierenden chemischen Substanz geeignet ist, wobei die Nanostruktur auf dem Boden oder einer Seitenwand der Kammer oder auf dem Boden oder einer Seitenwand des Kanals oder auf den Führungselektroden angeordnet ist.

8. Mikrowellenelektrode nach Anspruch 1, wobei
der Kanal eine Breite von 0,5 - 100 nm aufweist; und/oder
der Kanal eine Länge von 50 nm - 100 µm aufweist; und/oder
der Kanal eine Tiefe von 0 - 10 µm aufweist; und/oder
die erste Elektrode eine Dicke von 1- 1000 nm aufweist; und/oder
die zweite Elektrode eine Dicke von 1 - 1000 nm aufweist.

9. Mikrowellenelektrode nach Anspruch 1, weiterhin umfassend:
eine Passivierungsschicht, die auf der Fläche der ersten Elektrode und/oder der zweiten Elektrode angeordnet ist.

10. Mikrowellenelektroden-Anordnung, umfassend: die Mikrowellenelektrode nach irgendeinem der Ansprüche 1 bis 9.

11. Mikrowellenelektroden-Anordnung nach Anspruch 10, wobei die Mikrowellenelektroden-Anordnung mehrere Mikrowellenelektroden umfasst,
die mehreren Mikrowellenelektroden in einer elliptischen, einer kreisförmigen, einer ringförmigen, einer fächerförmigen, einer rechteckigen, einer quadratischen, einer Zickzack- oder einer Getriebeform oder als eine Matrix von Zeilen und Spalten oder als gewalzte Schichten angeordnet sind.

12. Mikrowellenelektroden-Anordnung nach Anspruch 11, wobei
mehr als eine Mikrowellenelektrode eine Führungselektrode teilen.

13. Sensorchip, umfassend: die Mikrowellenelektroden-Anordnung nach irgendeinem der Ansprüche 10 bis 12.

14. Sequenzierungssystem, umfassend: den Sensorchip nach Anspruch 13.

15. Verfahren zum Herstellen einer Mikrowellenelektrode, umfassend:
Bereitstellen einer Substratstruktur, umfassend ein Substrat mit einer Isolierschicht auf ihrer Fläche und eine erste Trägerelement-Materialschicht auf der Isolierschicht, wobei die erste Trägerelement-Materialschicht auf ihrer Seitenwand aufeinanderfolgend eine erste Elektrodenmaterialschicht, eine Opfermaterialschicht, eine zweite Elektrodenmaterialschicht und eine zweite Trägerelement-Materialschicht aufweist.
Strukturieren der ersten Trägerelement-Materialschicht, der ersten Elektrodenmaterialschicht, der Opfermaterialschicht, der zweiten Elektrodenmaterialschicht und der zweiten Trägerelement-Materialschicht zum Bilden einer oder mehrerer Kammer(n), eines ersten Trägerelements und zum Bilden einer ersten Elektrode, einer Opferschicht, einer zweiten Elektrode und eines zweiten Trägerelements, die aufeinanderfolgend auf der Seitenwand des ersten Trägerelements angeordnet sind;
Bilden von einer oder mehreren Führungselektrode(n) in der Kammer;
Entfernen der Opferschicht auf der Seitenwand des ersten Trägerelements zum Bilden eines Kanals zwischen der ersten Elektrode und der zweiten Elektrode,
wobei der Kanal wenigstens ein mit der Kammer in Verbindung stehendes Ende aufweist.

16. Verfahren nach Anspruch 15, wobei der Bereitstellungsschritt der Substratstruktur umfasst:
Bereitstellen eines Substrats mit einer Isolierschicht auf seiner Oberfläche;
Bilden einer ersten Trägerelement-Materialschicht auf einem Abschnitt der Isolierschicht;
Aufbringen einer ersten Elektrodenmaterialschicht zum Abdecken der oberen Fläche und einer Seitenwand der ersten Trägerelement-Materialschicht;
Entfernen der ersten Elektrodenmaterialschicht auf der oberen Fläche der ersten Trägerelement-Materialschicht;
Aufbringen einer Opfermaterialschicht zum Abdecken der oberen Fläche der ersten Trägerelement-Materialschicht, der oberen Fläche und einer Seitenwand der verbleibenden ersten Elektrodenmaterialschicht;
Entfernen der Opfermaterialschicht auf der oberen Fläche der ersten Trägerelement-Materialschicht und der oberen Fläche der verbleibenden ersten Elektrodenmaterialschicht;
Aufbringen einer zweiten Elektrodenmaterialschicht zum Abdecken der oberen Fläche der ersten Trägerelement-Materialschicht, oder oberen Fläche der verbleibenden ersten Elektrodenmaterialschicht und einer oberen Fläche und einer Seitenwand der verbleibenden Opfermaterialschicht,
Entfernen der zweiten Elektrodenmaterialschicht auf der oberen Fläche der ersten Trägerelement-Materialschicht, der oberen Fläche der verbleibenden ersten Elektrodenmaterialschicht und der oberen Fläche der verbleibenden Opfermaterialschicht;
Aufbringen einer zweiten Trägerelement-Materialschicht zum Abdecken der ersten Trägerelement-Materialschicht, der ersten Elektrodenmaterialschicht auf der Seitenwand der ersten Trägerelement-Materialschicht, der Opfermaterialschicht über der Seitenwand der ersten Trägerelement-Materialschicht, der zweiten Elektrodenmaterialschicht über der Seitenwand der ersten Trägerelement-Materialschicht und des Abschnitts, der nicht von der Isolierschicht abgedeckt ist;
Planarisieren der aufgebrachten zweiten Trägerelement-Materialschicht zum Freilegen der Opfermaterialschicht über der Seitenwand der ersten Trägerelement-Materialschicht.

17. Verfahren nach Anspruch 15, wobei das Verfahren vor dem Entfernen der Opferschicht weiterhin umfasst:
Bilden einer Passivierungsschicht auf einer Fläche wenigstens eines des ersten Trägerelements, des zweiten Trägerelements, der ersten Elektrode oder der zweiten Elektrode.

18. Verfahren nach Anspruch 15, wobei vor dem Entfernen der Opferschicht das Verfahren weiterhin umfasst:
Entfernen eines Abschnitts der Spitze des ersten Trägerelements und eines Abschnitts der Spitze des zweiten Trägerelements zum Freilegen der ersten Elektrode, eines Abschnitts der Opferschicht und eines Abschnitts der zweiten Elektrode;
Aufbringen einer Passivierungsschicht auf dem verbleibenden ersten Trägerelement, dem verbleibenden zweiten Trägerelement, dem freigelegten Abschnitt der ersten Elektrode, dem freigelegten Abschnitt der Opferschicht und dem freigelegten Abschnitt der zweiten Elektrode;
Planarisieren der aufgebrachten Passivierungsschicht zum Bilden einer Passivierungsschicht auf dem verbleibenden Abschnitt des ersten Trägerelements und des verbleibenden Abschnitts des zweiten Trägerelements und Freilegen der Opferschicht.

19. Verfahren nach Anspruch 15, wobei der Strukturierungsschritt umfasst:
Trennen der ersten Elektrodenmaterialschicht und/oder der zweiten Elektrodenmaterialschicht in mehrere Segmente derart, dass die jeweils gebildete erste Elektrode und/oder die zweite Elektrode mehrere voneinander getrennte Segmente umfassen.

20. Verfahren nach Anspruch 15, wobei das Verfahren weiterhin umfasst:
Bilden einer Nanostruktur, die zum Festsetzen eines Enzyms oder einer chemischen Substanz geeignet ist, das/die auf dem Boden oder einer Seitenwand der Kammer oder auf dem Boden oder einer Seitenwand des Kanals oder auf den Führungselektroden zu detektieren ist.

21. Verfahren nach Anspruch 15, wobei
der Kanal eine Breite von 0,5 - 100 nm aufweist und/oder
der Kanal eine Länge von 50 nm - 100 µm aufweist; und/oder
der Kanal eine Tiefe von 0 - 10 µm aufweist; und/oder
die erste Elektrode eine Dicke von 1 - 1000 nm aufweist; und/oder
die Opferschicht eine Dicke von 0,5 - 100 nm aufweist; und/oder
die zweite Elektrode eine Dicke von 1 - 1000 nm aufweist.

22. Verfahren nach Anspruch 15, wobei
das erste Trägerelement ein leitendes Element umfasst; und/oder
das zweite Trägerelement ein leitendes Element umfasst.

23. Verfahren zur Analyse einer chemischen Substanz, umfassend die Schritte:
(1) Bereitstellen der Mikrowellenelektrode nach irgendeinem der Ansprüche 1 - 9 oder der Mikrowellenelektroden-Anordnung nach irgendeinem der Ansprüche 10 - 12;
(2) Hinzufügen einer Reaktionslösung, die eine zu testende chemische Substanz enthält, zu der Mikrowellenelektrode oder der Mikrowellenelektroden-Anordnung und Unterziehen der Reaktionslösung einer Reaktion zum Produzieren eines geladenen Moleküls;
(3) Zulassen, dass das geladene Molekül unter der Wirkung der Führungselektrode und/oder hydromechanischen Wirkungen in den Kanal eintritt oder unter der Wirkung der Führungselektrode in dem Kanal angesammelt wird; und
(4) Identifizieren des Typs des geladenen Moleküls durch Verwenden der ersten Elektrode, der zweiten Elektrode und/oder der Führungselektrode und daher Erhalten der Informationen der zu testenden chemischen Substanz.

24. Verfahren nach Anspruch 23, wobei in Schritt (4) der Typ des geladenen Moleküls mit der ersten Elektrode, der zweiten Elektrode und/oder der Führungselektrode basierend auf einer oder mehreren Effekt(en) identifiziert wird, die aus einer Gruppe ausgewählt ist/sind, bestehend auf einem Oxidations-Reduktioneffekt, einem elektrischen Widerstandeffekt, einem Kapazitätseffekt, einem Feldeffekt und einem Tunneleffekt.

25. Verfahren zur Analyse eines Nukleinsäuremoleküls, umfassend die Schritte:
(1) Bereitstellen der Mikrowellenelektrode nach irgendeinem der Ansprüche 1 - 9 oder der Mikrowellenelektroden-Anordnung nach irgendeinem der Ansprüche 10 - 12;
(2) Festsetzen einer Polymerase (wie z. B. einer DNA-Polymerase oder einer RNA-Polymerase) in der Kammer oder dem Kanal der Führungselektrode der Mikrowellenelektrode oder der Mikrowellenelektroden-Anordnung oder darauf;
(3) Hinzufügen einer Reaktionslösung, die ein zu testendes Nukleinsäuremolekül, einen Primer und wenigstens ein (z. B. ein, zwei, drei oder vier) Deoxyribonukleosid-Triphosphat-Molekül (dNTP-Molekül) oder Nukleosid-Triphosphat-Molekül (NTP-Molekül) oder ein Analog davon, enthält, zu der Mikrowellenelektrode oder der Mikrowellenelektroden-Anordnung, wobei der Primer zu einer Teilsequenz des zu testenden Nukleinsäuremoleküls hybridisieren oder härten kann und jedes des wenigstens einen dNTP- oder NTP-Moleküls oder Analogs jeweils mit einem Markermolekül modifiziert wird; und später Hybridisieren des zu testenden Nukleinsäuremoleküls unter geeigneten Bedingungen mit dem Primer zum Bilden eines Komplexes;
(4) bei Vorhandensein der Polymerase als Katalysator, Integrieren entweder des markermolekül-modifizierten dNTP- oder NTP-Moleküls oder -Analogs in den Primer zum Bilden eines Erweiterungsprodukts, das zu den zu testenden Nukleinsäuremolekül komplementär ist, und Entfernen des Markermoleküls, das von dem dNTP- oder NTP-Molekül oder Analog getragen wird, das in den Primer integriert ist, um ein freies Markermolekül bereitzustellen, wobei das freie Markermolekül geladen wird;
(5) Zulassen, dass das freie Markermolekül unter der Wirkung der Führungselektrode und/oder eines hydromechanischen Effekts in den Kanal eintritt oder in dem Kanal unter der Wirkung der Führungselektrode angesammelt wird; das freie Markermolekül wird bevorzugt gesteuert, um durch seine elektrische Polarität oder Freisetzungsordnung in unterschiedliche Mikrowellenkanäle einzutreten oder sich darin anzusammeln;
(6) Identifizieren des Typs des freien Markermoleküls unter Verwenden der ersten Elektrode und der zweiten Elektrode; und weiterhin Identifizieren des Typs des dNTP- oder NTP-Moleküls oder -Analogs, das in den Primer integriert ist, nach der Entsprechung zwischen dem Markermolekül und dem dNTP- oder NTP-Molekül oder -Analog; und weiterhin Bestimmen der Basis an der entsprechenden Position des zu testenden Nuklearsäuremoleküls nach dem Grundsatz der komplementären Basispaarung; und
(7) Wiederholen der Schritte (4), (5) und (6) bis die Erweiterung des Komplexes beendet ist.

26. Verfahren nach Anspruch 25, wobei das freie Markermolekül eine aktive Redoxsubstanz sein kann, die in einer kreisförmigen Redoxreaktion reaktiv ist oder in eine aktive Redoxsubstanz konvertiert werden kann, die in einer kreisförmigen Redoxreaktion reaktiv ist; die aktive Redoxsubstanz bevorzugt einer kreisförmigen Redoxreaktion zwischen der ersten Elektrode und der zweiten Elektrode unterzogen werden kann, was in einen detektierbaren Strom resultiert.

27. Verfahren nach Anspruch 25, wobei die Reaktionslösung weiterhin eine Phosphatase umfasst.

28. Verfahren nach Anspruch 25, wobei in Schritt (4) das freie Markermolekül bei Vorhandensein einer Phosphatase dephosphyriliert wird.

29. Verfahren nach Anspruch 25, wobei die von dem freien Molekül getragene Charge durch Auswählen eines Markermoleküls derart angepasst wird, dass die Migrationsgeschwindigkeit des freien Markermoleküls unter der Wirkung der Führungselektrode angepasst wird.

30. Verfahren nach Anspruch 25, wobei in Schritt (1) die Polymerase auf einer Isolierschicht auf dem Boden der Kammer oder des Kanals festgesetzt wird oder auf der Führungselektrode festgesetzt wird; die Polymerase bevorzugt an einer Stelle in der Nähe des Endes des Kanals am Boden der Kammer festgesetzt wird.

31. Verfahren nach Anspruch 30, wobei
eine funktionalisierbare Region und/oder eine molekülbindende Region weiterhin zwischen der Isolierschicht und der Polymerase bereitgestellt wird;
die funktionalisierbare Region bevorzugt Siliziumdioxid, Hafniumdioxid, Aluminiumdioxid, Tantaldioxid und/oder Zirkoniumoxid umfasst; weiter bevorzugt das funktionalisierbare Material mit einem Verbindungsmolekül funktionalisiert wird, das aus einer Gruppe ausgewählt wird, bestehend aus: Silikan (z. B. Aminopropyltriethoxysilan), Thiol (-SH), Disulfid (-S-S-), Isothiocyanat, Alken und Alkyn;
die molekülbindende Region bevorzugt ein Sondenmolekül umfasst; die Molekülsonde bevorzugt z. B. aus einer Gruppe ausgewählt wird, bestehend aus einem Biotin, einem Avidin, einem Antikörper, einem Antigen, einem Rezeptor, einem Liganden, einer DNA-Sequenz, einer RNA-Sequenz, einem Protein und einem Liganden davon.

32. Verfahren nach Anspruch 25, wobei in Schritt (6) der Typ des freien Markermoleküls durch einen oder mehrere Oxidations-Reduktionseffekt(e), elektrische(n) Widerstandseffekt(e), Kapazitätseffekt(e), Feldeffekt(e) und Tunneleffekt(e) identifiziert wird.

## Revendications

1. Électrode de micropuits, comprenant :
un substrat et une couche d'isolation sur une surface du substrat ;
une ou plusieurs premières électrodes ;
une ou plusieurs deuxièmes électrodes agencées chacune à l'opposé d'une première électrode, dans laquelle un canal est défini et situé entre chaque première électrode et deuxième électrode qui lui est opposée, et le canal comporte au moins une extrémité en communication avec une chambre ; et
une ou plusieurs électrodes de guidage situées dans la chambre,
dans laquelle la première électrode, la deuxième électrode et les électrodes de guidage sont situées sur une surface de la couche d'isolation éloignée du substrat.

2. Électrode de micropuits selon la revendication 1, comprenant en outre :
un premier élément de support pour supporter lesdites une ou plusieurs électrodes.

3. Électrode de micropuits selon la revendication 1, dans laquelle
l'électrode de micropuits comprend une pluralité de premières électrodes et une pluralité de premiers éléments de support, chaque électrode étant supportée par un premier élément de support correspondant ; et/ou
l'électrode de micropuits comprend une pluralité de deuxièmes électrodes et une pluralité de deuxièmes éléments de support, chaque deuxième électrode étant supportée par un deuxième élément de support correspondant.

4. Électrode de micropuits selon la revendication 1, dans laquelle
au moins une électrode parmi la première électrode et la deuxième électrode comprend une pluralité de segments séparés les uns des autres.

5. Électrode de micropuits selon la revendication 4, comprenant en outre :
une pluralité de premiers éléments de support, chaque segment de la première électrode étant supporté par un premier élément de support correspondant ; et/ou
une pluralité de deuxièmes éléments de support, chaque segment de la deuxième électrode étant supporté par un deuxième élément de support correspondant.

6. Électrode de micropuits selon la revendication 5, dans laquelle
le premier élément de support est un élément conducteur ; et/ou
le deuxième élément de support est un élément conducteur.

7. Électrode de micropuits selon la revendication 1, comprenant en outre :
une nanostructure capable d'immobiliser une enzyme ou une substance chimique à détecter, dans laquelle la nanostructure est située sur le fond ou sur une paroi latérale de la chambre, ou sur le fond ou sur une paroi latérale du canal, ou sur les électrodes de guidage.

8. Électrode de micropuits selon la revendication 1, dans laquelle
le canal présente une largeur de 0,5 à 100 nm ; et/ou
le canal présente une longueur de 50 nm à 100 µm ; et/ou
le canal présente une profondeur de 0 à 10 µm ; et/ou
la première électrode présente une épaisseur de 1 à 1.000 nm ; et/ou
la deuxième électrode présente une épaisseur de 1 à 1.000 nm.

9. Électrode de micropuits selon la revendication 1, comprenant en outre :
une couche de passivation située sur la surface de la première électrode et/ou de la deuxième électrode.

10. Réseau d'électrodes de micropuits comprenant : l'électrode de micropuits selon l'une quelconque des revendications 1 à 9.

11. Réseau d'électrodes de micropuits selon la revendication 10, dans lequel le réseau d'électrodes de micropuits comprend une pluralité d'électrodes de micropuits,
les électrodes de la pluralité d'électrodes de micropuits sont agencées sous forme d'une ellipse, d'un cercle, d'un anneau, d'un éventail, d'un rectangle, d'un carré, d'un zigzag ou d'un engrenage, ou sous forme d'une matrice de lignes et de colonnes, ou sous forme de couches stratifiées.

12. Réseau d'électrodes de micropuits selon la revendication 11, dans lequel plusieurs électrodes de micropuits partagent une électrode de guidage.

13. Puce de capteur comprenant : le réseau d'électrodes de micropuits selon l'une quelconque des revendications 10 à 12.

14. Système de séquençage comprenant : la puce de capteur selon la revendication 13.

15. Procédé de fabrication d'une électrode de micropuits, comprenant :
la fourniture d'une structure de substrat comprenant un substrat avec une couche d'isolation sur sa surface et une première couche de matériau d'élément de support sur la couche d'isolation, dans lequel la première couche de matériau d'élément de support comporte successivement sur sa paroi latérale une première couche de matériau d'électrode, une couche de matériau sacrificiel, une deuxième couche de matériau d'électrode et une deuxième couche de matériau d'élément de support ;
la structuration de la première couche de matériau d'élément de support, de la première couche de matériau d'électrode, de la couche de matériau sacrificiel, de la deuxième couche de matériau d'électrode et de la deuxième couche de matériau d'élément de support pour former une ou plusieurs chambres et un premier élément de support, et pour former une première électrode, une couche sacrificielle, une deuxième électrode et un deuxième élément de support situés successivement sur la paroi latérale du premier élément de support ;
la formation d'une ou plusieurs électrodes de guidage dans la chambre ;
l'élimination de la couche sacrificielle sur la paroi latérale du premier élément de support pour former un canal entre la première électrode et la deuxième électrode,
dans lequel le canal comporte au moins une extrémité en communication avec la chambre.

16. Procédé selon la revendication 15, dans lequel l'étape de fourniture de la structure de substrat comprend :
la fourniture d'une couche d'isolation sur la surface d'un substrat ;
la formation d'une première couche de matériau d'élément de support sur une portion de la couche d'isolation ;
le dépôt d'une première couche de matériau d'électrode pour recouvrir la surface supérieure et une paroi latérale de la première couche de matériau d'élément de support ;
l'élimination de la première couche de matériau d'électrode sur la surface supérieure de la première couche de matériau d'élément de support ;
le dépôt d'une couche de matériau sacrificiel pour recouvrir la surface supérieure de la première couche de matériau d'élément de support, la surface supérieure et une paroi latérale de la première couche de matériau d'électrode restante ;
l'élimination de la couche de matériau sacrificiel sur la surface supérieure de la première couche de matériau d'élément de support et sur la surface supérieure de la première couche de matériau d'électrode restante ;
le dépôt d'une deuxième couche de matériau d'électrode pour recouvrir la surface supérieure de la première couche de matériau d'élément de support, la surface supérieure de la première couche de matériau d'électrode restante et une surface supérieure et une paroi latérale de la couche de matériau sacrificiel restante ;
l'élimination de la deuxième couche de matériau d'électrode sur la surface supérieure de la première couche de matériau d'élément de support, la surface supérieure de la première couche de matériau d'électrode restante, et la surface supérieure de la couche de matériau sacrificiel restante ;
le dépôt d'une deuxième couche de matériau d'élément de support pour recouvrir la première couche de matériau d'élément de support, la première couche de matériau d'électrode sur la paroi latérale de la première couche de matériau d'élément de support, la couche de matériau sacrificiel sur la paroi latérale de la première couche de matériau d'élément de support, la deuxième couche de matériau d'électrode sur la paroi latérale de la première couche de matériau d'élément de support, et la portion qui n'est pas recouverte par la couche d'isolation ;
l'aplanissement de la deuxième couche de matériau d'élément de support déposée pour exposer la couche de matériau sacrificiel sur la paroi latérale de la première couche de matériau d'élément de support.

17. Procédé selon la revendication 15 dans lequel, avant l'élimination de la couche sacrificielle, le procédé comprend en outre :
la formation d'une couche de passivation sur une surface d'au moins un élément parmi le premier élément de support, le deuxième élément de support, la première électrode et la deuxième électrode.

18. Procédé selon la revendication 15 dans lequel, avant l'élimination de la couche sacrificielle, le procédé comprend en outre :
l'élimination d'une portion du dessus du premier élément de support et d'une portion du dessus du deuxième élément de support pour exposer une portion de la première électrode, une portion de la couche sacrificielle et une portion de la deuxième électrode ;
le dépôt d'une couche de passivation sur le premier élément de support restant, le deuxième élément de support restant, la portion exposée de la première électrode, la portion exposée de la couche sacrificielle et la portion exposée de la deuxième électrode ;
l'aplanissement de la couche de passivation déposée pour former une couche de passivation sur la portion restante du premier élément de support et la portion restante du deuxième élément de support, et pour exposer la couche sacrificielle.

19. Procédé selon la revendication 15, dans lequel l'étape de structuration comprend :
la séparation de la première couche de matériau d'électrode et/ou de la deuxième couche de matériau d'électrode en une pluralité de segments, de telle sorte que la première électrode et/ou la deuxième électrode formées comprennent chacune une pluralité de segments séparés les uns des autres.

20. Procédé selon la revendication 15, dans lequel le procédé comprend en outre :
la formation d'une nanostructure capable d'immobiliser une enzyme ou une substance chimique à détecter sur le fond ou sur une paroi latérale de la chambre, ou sur le fond ou sur une paroi latérale du canal, ou sur les électrodes de guidage.

21. Procédé selon la revendication 15, dans lequel
le canal présente une largeur de 0,5 à 100 nm ; et/ou
le canal présente une longueur de 50 nm à 100 µm ; et/ou
le canal présente une profondeur de 0 à 10 µm ; et/ou
la première électrode présente une épaisseur de 1 à 1.000 nm ; et/ou
la couche sacrificielle présente une épaisseur de 0,5 à 100 nm ; et/ou
la deuxième électrode présente une épaisseur de 1 à 1.000 nm.

22. Procédé selon la revendication 15, dans lequel
le premier élément de support comprend un élément conducteur ; et/ou
le deuxième élément de support comprend un élément conducteur.

23. Procédé d'analyse d'une substance chimique comprenant les étapes suivantes :
(1) fourniture de l'électrode de micropuits selon l'une quelconque des revendications 1 à 9 ou du réseau d'électrodes de micropuits selon l'une quelconque des revendications 10 à 12 ;
(2) ajout d'une solution de réaction contenant une substance chimique à tester à l'électrode de micropuits ou au réseau d'électrodes de micropuits, et soumission de la solution de réaction à une réaction pour produire une molécule chargée ;
(3) habilitation de l'entrée de la molécule chargée dans le canal sous l'action de l'électrode de guidage et/ou d'un effet hydromécanique, ou de son accumulation dans le canal sous l'action de l'électrode de guidage ; et
(4) identification du type de la molécule chargée en utilisant la première électrode, la deuxième électrode et/ou l'électrode de guidage, et par conséquent obtention de l'information concernant la substance chimique à tester.

24. Procédé selon la revendication 23, dans lequel à l'étape (4), le type de la molécule chargée est identifié avec la première électrode, la deuxième électrode et/ou l'électrode de guidage sur base d'un ou plusieurs effets sélectionnés parmi le groupe constitué par un effet d'oxydoréduction, un effet de résistance électrique, en effet capacitif, un effet de champ et un effet tunnel.

25. Procédé d'analyse d'une molécule d'acide nucléique, comprenant les étapes suivantes :
(1) fourniture de l'électrode de micropuits selon l'une quelconque des revendications 1 à 9 ou du réseau d'électrodes de micropuits selon l'une quelconque des revendications 10 à 12 ;
(2) immobilisation d'une polymérase (telle qu'une ADN polymérase ou une ARN polymérase) dans la chambre, dans le canal ou sur l'électrode de guidage de l'électrode de micropuits ou du réseau d'électrodes de micropuits ;
(3) ajout sur l'électrode de micropuits ou sur le réseau d'électrodes de micropuits d'une solution de réaction contenant une molécule d'acide nucléique à tester, une amorce, et au moins une (par exemple une, deux, trois ou quatre) molécule de désoxyribonucléoside triphosphate (dNTP) ou de nucléoside triphosphate (NTP), ou une molécule analogue à celles-ci, dans lequel l'amorce peut s'hybrider ou s'anneler avec une séquence partielle de la molécule d'acide nucléique à tester, et chacune desdites au moins une molécule dNTP, NTP ou analogue est modifiée avec une molécule d'étiquetage, respectivement ; et ensuite, sous une condition adaptée, hybridation de la molécule d'acide nucléique à tester avec l'amorce pour former un complexe ;
(4) en présence de la polymérase comme catalyseur, incorporation d'une molécule parmi une molécule dNTP, NTP ou analogue modifiée par la molécule d'étiquetage dans l'amorce pour former un produit d'extension complémentaire de la molécule d'acide nucléique à tester, et élimination de la molécule d'étiquetage portée par la molécule dNTP, NTP ou analogue incorporée dans l'amorce pour fournir une molécule d'étiquetage libre, dans lequel la molécule d'étiquetage libre est chargée ;
(5) habilitation de l'entrée de la molécule d'étiquetage libre dans le canal sous l'action de l'électrode de guidage et/ou d'un effet hydromécanique, ou de son accumulation dans le canal sous l'action de l'électrode de guidage ; la molécule d'étiquetage libre étant de préférence contrôlée pour entrer ou s'accumuler dans différents canaux d'électrode de micropuits par sa polarité électrique ou son ordre de libération ;
(6) identification du type de la molécule d'étiquetage libre en utilisant la première électrode et la deuxième électrode ; et ensuite identification du type de la molécule dNTP, NTP ou analogue incorporée dans l'amorce conformément à la correspondance entre la molécule d'étiquetage et la molécule dNTP, NTP ou analogue ; et ensuite détermination de la base à la position correspondante de la molécule d'acide nucléique à tester, conformément à l'appariement des bases par principe de complémentarité ; et
(7) répétition des étapes (4), (5) et (6) jusqu'à ce que l'extension du complexe soit terminée.

26. Procédé selon la revendication 25, dans lequel la molécule d'étiquetage libre peut être une substance active redox qui est réactive dans une réaction redox circulaire, ou qui peut être convertie en une substance active redox qui est réactive dans une réaction redox circulaire ; la substance active redox pouvant préférablement être soumise à une réaction redox circulaire entre la première électrode et la deuxième électrode, ce qui résulte en un courant détectable.

27. Procédé selon la revendication 25, dans lequel la solution de réaction comprend en outre une phosphatase.

28. Procédé selon la revendication 25 dans lequel, à l'étape (4), la molécule d'étiquetage libre est déphosphorylée en présence d'une phosphatase.

29. Procédé selon la revendication 25, dans lequel la charge portée par la molécule d'étiquetage libre est ajustée en sélectionnant une molécule d'étiquetage de manière à ajuster la vitesse de migration de la molécule d'étiquetage libre sous l'action de l'électrode de guidage.

30. Procédé selon la revendication 25, dans lequel à l'étape (1), la polymérase est immobilisée sur une couche isolée au fond de la chambre ou du canal, ou est immobilisée sur l'électrode de guidage ; de préférence, la polymérase est immobilisée à un endroit proche de l'extrémité du canal au fond de la chambre.

31. Procédé selon la revendication 30, dans lequel
une région fonctionnalisable et/ou une région de liaison moléculaire est en outre pourvue entre la couche isolée et la polymérase ;
de préférence, la région fonctionnalisable comprend du dioxyde de silicium, de l'oxyde d'hafnium, de l'oxyde d'aluminium, de l'oxyde de tantale et/ou de l'oxyde de zirconium ; de préférence encore, le matériau fonctionnalisable est fonctionnalisé avec une molécule de liaison sélectionnée parmi le groupe constitué par : les silanes (par exemple l'aminopropyl-triéthoxysilane), les thiols (-SH), les disulfures (-S-S-), les isothiocyanates, les alcènes et les alcynes ;
de préférence, la région de liaison moléculaire comprend une molécule sonde ; de préférence, la molécule sonde est par exemple sélectionnée parmi le groupe constitué par la biotine, l'avidine, un anticorps, un antigène, un récepteur, un ligand, une séquence d'ADN, une séquence d'ARN, une protéine et un ligand de celle-ci.

32. Procédé selon la revendication 25 dans lequel, à l'étape (6), le type de la molécule d'étiquetage libre est identifié par un ou plusieurs effets parmi un effet d'oxydoréduction, un effet de résistance électrique, un effet capacitif, un effet de champ et un effet tunnel.
